(19) **Europäisches Patentamt / European Patent Office / Office européen des brevets**

(11) **EP 3 939 512 A1**

(12) # EUROPEAN PATENT APPLICATION

(43) Date of publication:
**19.01.2022 Bulletin 2022/03**

(21) Application number: **21172134.5**

(22) Date of filing: **03.06.2016**

(51) International Patent Classification (IPC):
**A61B 6/12** (2006.01)    **A61B 8/12** (2006.01)
**A61N 5/10** (2006.01)    **A61B 6/03** (2006.01)
**A61N 5/00** (2006.01)    **A61B 18/00** (2006.01)

(52) Cooperative Patent Classification (CPC):
**A61B 6/12; A61B 6/032; A61B 8/12; A61N 5/1027; A61N 5/1049;** A61B 2018/00547; A61N 2005/1058

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2015 US 201562170219 P**

(62) Document number(s) of the earlier application(s) in accordance with Art. 76 EPC:
**16804547.4 / 3 302 280**

(71) Applicant: **Memorial Sloan-Kettering Cancer Center**
**New York, NY 10065 (US)**

(72) Inventors:
  • **Cohen, Gil'ad N.**
    **Tenafly, NJ 07670 (US)**
  • **ZELEFSKY, Michael J.**
    **New York, 10024 (US)**
  • **HU, Yu-Chi**
    **New York, 10021 (US)**
  • **XIONG, Jian-Ping**
    **Plainsboro, NJ 08536 (US)**
  • **ZAIDER, Marco**
    **Hastings on Hudson, NY 10706 (US)**
  • **MAGERAS, Gikas**
    **White Plains, NY 10604 (US)**
  • **NASSER, Nicola J.**
    **2020010 Sheefamre (IL)**
  • **AMOLS, Howard I.**
    **Allentown, PA 18104 (US)**

(74) Representative: **Maiwald Patent- und Rechtsanwaltsgesellschaft mbH**
**Elisenhof**
**Elisenstraße 3**
**80335 München (DE)**

Remarks:
• This application was filed on 04.05.2021 as a divisional application to the application mentioned under INID code 62.
• Claims filed after the date of receipt of the divisional application (Rule 68(4) EPC).

(54) **COMPUTER-ACCESSIBLE MEDIUM, METHOD AND SYSTEM FOR INTRAOPERATIVE CONE BEAM CT FOR LOW-DOSE-RATE PROSTATE BRACHYTHERAPY**

(57) An exemplary system, method and computer-accessible medium for determining a location a radioactive seed(s) within a body, can include, for example receiving first imaging information of the radioactive seed(s) within the body based on a first imaging modality, receiving second imaging information of the radioactive seed(s) within the body based on a second imaging modality, and determining the location of the radioactive seed(s) within the body based on the first and second imaging information. The first imaging modality can be computed tomography, and the second imaging modality can be an ultrasound, which can be a transrectal ultrasound. Third information related to a seed implantation plan for a patient(s) can be received.

Figure 3B

**Description**

CROSS-REFERENCE TO RELATED APPLICATION(S)

This application relates to and claims priority from U.S. Patent Application No. 62/170,219, filed on June 3, 2015, the entire disclosure of which is incorporated herein by reference.

FIELD OF THE DISCLOSURE

The present disclosure relates generally to radiation seed implantation, and more specifically, to exemplary embodiments of exemplary system, method, computer-accessible medium and apparatus for fast radioactive seed localization in intraoperative cone beam computed tomography ("CT").

BACKGROUND INFORMATION

Radiation therapy can be an important treatment modality for cancer. Such therapy can be delivered as external beam radiation or brachytherapy. Brachytherapy can usually be performed under imaging, such as, e.g., transrectal ultrasound ("TRUS") or Magnetic Resonance Imaging ("MRI").

Prostate brachytherapy is used to treat prostate cancer, and involves an insertion of radioactive sources inside the prostate. Low-Dose-Rate ("LDR") brachytherapy involves an implantation of one or more radioactive sources permanently inside the prostate, and High-Dose-Rate ("HDR") brachytherapy involves temporary insertion of a radioactive source into the prostate. Both LDR and HDR brachytherapy involve the insertion of needles or catheters into the prostate gland, after which a plan is developed to determine the location of seed insertion in LDR brachytherapy, or the location and timing of the radioactive seed in HDR brachytherapy *(e.g.,* dwell time).

The determination of the location of the brachytherapy needles/catheters inside the prostate is important in order to generate a reliable plan. This plan ensures that the radiation is delivered properly to cover the prostate, and/or dose escalate the tumor, and keep organs at risk - such as the rectum and urethra - under specific tolerance limits.

The determination of the location of the brachytherapy needles/catheters inside the prostate is performed at the completion of the needle insertion, likely manually performed, on each slice of the images captured by the TRUS. Current methods may be reliable, but still there are human-type errors in the localization of each needle on each ultrasound ("US") image section, and the procedure is time consuming in the operation room, as it takes up to 20 minutes of operation room time in experienced hands. Many times manipulation of the needles after their insertion is performed to discriminate between possible needle locations or artifacts, which increases the trauma to the prostate gland.

Prostate seed implantation is the current standard of care for prostate cancer. However, currently the implants can only be evaluated *(e.g.,* for dosimetric outcome) to determine their quality, and specifically how well the intended dose was delivered to the prostate well after the implantation procedure has been completed. In many cases, this quality control ("QC") evaluation is performed several hours after the procedure. In some cases, the QC is performed as long as 30 days after the implantation procedure. However, there are several recognized limitations with the way prostate seed implantations are performed today. For example, the standard procedure used does not provide an opportunity to modify the implant during implantation if changes need to be made to correct any deficiencies due to the seed placement and/or if an area is devoid of the needed dose. Previously, a mechanism to provide real time information regarding the exact coordinates of the implanted seed locations, and radiation doses delivered to the prostate, during the procedure has not been available, and as a result, it has not been possible to optimally "fix" a suboptimal seed implant.

Deviations in the locations of the implanted seeds from their planned positions can result in deviations in the dose delivered to the target. Therefore, providing an opportunity for evaluation of the quality of the implant during the actual implant procedure can benefit from the ability to detect whether the actual locations of the deposited seeds within the gland deviated from the original planned coordinates of the seeds. Even in expert hands, approximately 20% of implants may not actually deliver the full intended prescription dose to the prostate due to the variability of seed placement. Thus an intraoperative evaluation can provide an enormous benefit for the patient. In the typical scenario, once the QC assessment demonstrates that an implant is deficient in terms of delivering the intended dose to the target, there are two implications: (i) difficulty in mending a deficient implant and (ii) regulatory compliance. For the latter, if an implant is grossly deficient delivering less than 80% of the intended dose, it may be considered a medical event. For both instances, whether an implant may only be slightly deficient or grossly deficient, the only way to fix a treatment after it is completed would be to supplement it with another treatment. Yet additional treatment may not always be feasible or desirable.

The use of HDR brachytherapy is proliferating rapidly, and with the availability of sophisticated imaging and treatment planning systems, HDR treatments are becoming increasingly complex. These developments can result in an increased reliance on treatment-planning systems, because adequate methods for independent dose calculations may not be readily available. QA for HDR treatment planning systems, as well as individual treatment plans, can be a legal requirement. The NRC *(see, e.g.,* Reference 1) defines dose errors greater than 10% as recordable events and errors greater than 20% as misadministrations. AAPM TG-40 recommends the agreement between the delivered and prescribed doses should be within 15%, and that at least one dose-to-point calculation be performed (e.g., although a comparison of total dwell time with classical mg•h tables can be acceptable for some clinical implants). *(See, e.g.,* Reference 2). It can also be needed that the dose calculations be checked before 50% of the dose can be delivered. In practice, this requirement, written with LDR implants in mind, means that for HDR treatments, secondary verification should be done before treatment. In many clinical practices, independent checks of dose calculations to these levels of accuracy are not always feasible.

The QA of treatment planning has been the subject of several communications, and was extensively reviewed in AAPM TG-59 report. *(See, e.g.,* Reference 3). Generally, techniques described in these reports *(see e.g.,* References 4-7) apply empiric relations with a stated accuracy of the order of 10% to a particular application, which, according to a strict interpretation of NRC regulations, cannot "detect" misadministrations. HDR procedures generally fall into two categories. The first category employs fixed-geometry applicators. While many applicators are available, the exemplary discussion is based on single-channel intravaginal tandem, endobronchial and esophageal applicators, GYN ring applicators, and Harrison-Anderson-Mick ("HAM") applicator used for intraoperative radiotherapy ("IORT"). These applicators, and many others, have been reviewed extensively. *(See e.g.,* Reference 10). Most of the treatments in this category, IORT especially, require that a treatment plan be generated and implemented quickly and reliably while the patient is waiting in the procedure/operating room. The second category consists of generalized geometry template-based HDR procedures using computed tomography ("CT") or ultrasound US modalities for treatment planning, such as, for example, HDR treatment of the prostate. The challenge can be to perform an accurate dose-to-point calculation.

Thus, it may be beneficial to provide an exemplary system, method, computer-accessible medium and apparatus, which can overcome at least some of the deficiencies described herein above.

## SUMMARY OF EXEMPLARY EMBODIMENTS

An exemplary system, method and computer-accessible medium for determining a location a radioactive seed(s) within a body, can include, for example, receiving first imaging information of the radioactive seed(s) within the body based on a first imaging modality, receiving second imaging information of the radioactive seed(s) within the body based on a second imaging modality, and determining the location of the radioactive seed(s) within the body based on the first and second imaging information. The first imaging modality can be computed tomography, and the second imaging modality can be an ultrasound, which can be a transrectal ultrasound. Third information related to a seed implantation plan for a patient(s) can be received.

In some exemplary embodiments of the present disclosure, the radioactive seed(s) can include a plurality of radioactive seeds, and a first location of each radioactive seed of the radioactive seeds in the body of the patient(s) can be compared with a second proposed location in the body of the patient(s) for each radioactive seed contained in the third information. The first imaging information can be in a first imaging space, and the second imaging information can be in a second imaging space, and the first imaging information can be transformed from the first imaging space to the second imaging space.

A further exemplary embodiment of the present disclosure can include an exemplary system, method and computer-accessible medium for controlling or modifying a radiation therapy treatment plan(s) associated with a patient, which can include, for example, receiving first information related to a first location of a needle(s) in a needle insertion template(s), receiving second information related to a second location of the needle(s) in a body of the patient, and controlling or modifying the radiation therapy treatment plan(s) based on the first and second information. In some exemplary embodiments of the present disclosure, third information related to a third location of a further needle(s) in the needle insertion template(s) can be received, fourth information related to a fourth location of the further needle(s) in the body of the patient can be received, and the radiation therapy treatment plan(s) can be controlled or modified based on the first, second, third and fourth information.

In certain exemplary embodiments of the present disclosure, the radiation therapy treatment plan(s) can be modified by by determining a location of an insertion of a further needle(s) in the body of the patient. The first information can be determined by receiving third information related to light received by a light emitting diode (LED) detector(s), receiving fourth information related to an absence of the light at the LED detector(s) and determining the first location of the needle(s) in the needle insertion template(s) based on the fourth information. The needle insertion template(s) can include a plurality of needle insertion holes, where the LED detector(s) can be associated with a particular one of the needle insertion holes.

In some exemplary embodiments of the present disclosure, the second information can be determined by receiving third information related to an ultrasound (US) image(s) without the needle(s) contained therein, receiving fourth information related to further US image(s) having the needle(s) contained therein, and determining the second information based on the third and fourth information. The second information can be determined by digitally subtracting the third information from the fourth information. The needle insertion template(s) can include a plurality of needle insertion holes. In certain exemplary embodiments of the present disclosure, the radiation therapy treatment plan(s) can be generated.

Still a further exemplary embodiment of the present disclosure can include an exemplary system, method and computer-accessible medium for verifying a radiation therapy treatment plan(s), which can include, for example, receiving first information related to a treatment geometry(ies) associated with the radiation therapy treatment plan(s), receiving second information related to a dose calculation(s) based on the treatment geometry(ies), and verifying the radiation therapy treatment plan(s) based on the first and second information. The treatment geometry(ies) and/or the dose calculations(s) can be determined. The treatment geometry(ies) can be determined based on a plurality of dwell positions, which can be correct dwell positions.

In some exemplary embodiments of the present disclosure, each of the dwell positions can include a nominal dwell time and/or a set of coordinates in a patient's coordinate space. The set of coordinates can be defined by a plurality of active channels. The set of coordinates can be approximated using a user input.

In certain exemplary embodiments of the present disclosure, the dose calculation can include a dose-to-point calculation(s). The dose calculation(s) can include a summation of dose contributions from all dwell positions based on a two-dimensional dose table.

These and other objects, features and advantages of the exemplary embodiments of the present disclosure will become apparent upon reading the following detailed description of the exemplary embodiments of the present disclosure, when taken in conjunction with the appended claims.

## BRIEF DESCRIPTION OF THE DRAWINGS

Further objects, features and advantages of the present disclosure will become apparent from the following detailed description taken in conjunction with the accompanying Figures showing illustrative embodiments of the present disclosure, in which:

Figure 1 is an exemplary diagram of a patient during a prostate brachytherapy procedure according to an exemplary embodiment of the present disclosure;

Figures 2 is an exemplary diagram illustrating an exemplary ultrasound machine which has multiple ultrasound probes aligned one beside the other according to an exemplary embodiment of the present disclosure;

Figures 3A and 3B are exemplary diagrams of the prostate brachytherapy procedure and the exemplary automatic needle localization and detection system according to an exemplary embodiment of the present disclosure;

Figure 4A is an exemplary diagram of an exemplary template grid for the prostate brachytherapy according to an exemplary embodiment of the present disclosure;

Figure 4B is an exemplary diagram of one unit of the template grid from Figure 4A according to an exemplary embodiment of the present disclosure;

Figure 4C is an exemplary diagram of a light emitting diode and a light detector according to an exemplary embodiment of the present disclosure;

Figures 5A-5C are exemplary diagrams illustrating an exemplary unit of the template grid from Figure 4A according to an exemplary embodiment of the present disclosure;

Figure 6 is an exemplary diagram illustrating the exemplary template grid connected to an exemplary computer system according to an exemplary embodiment of the present disclosure;

Figure 7 is an exemplary image of an intraoperative apparatus and an ultrasound probe placed inside of a patient according to an exemplary embodiment of the present disclosure;

Figure 8A is an exemplary diagram of an ultrasound probe according to an exemplary embodiment of the present disclosure;

Figure 8B is an exemplary image of an ultrasound probe inserted into a patient according to an exemplary embodiment of the present disclosure;

Figure 8C is an exemplary schematic diagram of an ultrasound probe according to an exemplary embodiment of the present disclosure;

Figure 8D is an exemplary image of an ultrasound probe according to an exemplary embodiment of the present disclosure;

Figure 9 is a set of exemplary images of an ultrasound probe inserted into a patient according to an exemplary embodiment of the present disclosure;

Figure 10 is an exemplary image of multiple seeds implanted into a patient according to an exemplary embodiment of the present disclosure;

Figure 11 is an exemplary image of a standard HAM applicator;

Figure 12 is an exemplary image of a breast applicator according to an exemplary embodiment of the present disclosure;

Figure 13 is an exemplary diagram of a custom treatment plan according to an exemplary embodiment of the present disclosure;

Figure 14 is an exemplary IORT worksheet;

Figure 15 is an exemplary diagram of a planar geometer of an IORT HAM applicator according to an exemplary embodiment of the present disclosure;

Figure 16 is an exemplary image of a lateral CT scout view of an implant geometry according to an exemplary embodiment of the present disclosure;

Figure 17 is an exemplary image of an axial CT image of the pelvis according to an exemplary embodiment of the present disclosure;

Figure 18 is an exemplary diagram of a lateral view of a prostate template-based implant according to an exemplary embodiment of the present disclosure;

Figure 19A is an exemplary histogram showing changes in does-to-point calculations according to an exemplary embodiment of the present disclosure;

Figure 19B is a further exemplary histogram showing changes in the does-to-point calculations according to another exemplary embodiment of the present disclosure;

Figure 20A is an exemplary flow diagram of an exemplary method for determining the location of a radioactive seed within the body according to an exemplary embodiment of the present disclosure;

Figure 20B is an exemplary flow diagram of an exemplary method for controlling or modifying at least one radiation therapy treatment plan associated with a patient according to an exemplary embodiment of the present disclosure;

Figure 20C is an exemplary flow diagram of an exemplary method for verifying a radiation therapy treatment plan according to an exemplary embodiment of the present disclosure; and

Figure 21 is an illustration of an exemplary block diagram of an exemplary system in accordance with certain exemplary embodiments of the present disclosure.

Throughout the drawings, the same reference numerals and characters, unless otherwise stated, are used to denote like features, elements, components or portions of the illustrated embodiments. Moreover, while the present disclosure will now be described in detail with reference to the figures, it is done so in connection with the illustrative embodiments and is not limited by the particular embodiments illustrated in the figures and the appended claims.

## DETAILED DESCRIPTION OF EXEMPLARY EMBODIMENTS

The exemplary method/apparatus is described herein in relation to a method for real-time automatic localization and labeling of prostate brachytherapy needles under imaging. However, it should be understood that the exemplary method apparatus can be further applied and/or used to localize any catheter/needle or foreign body being inserted into the body by having first imaging done, having a needle inserted, repeating the imaging, and determining the location of the needle inside the body.

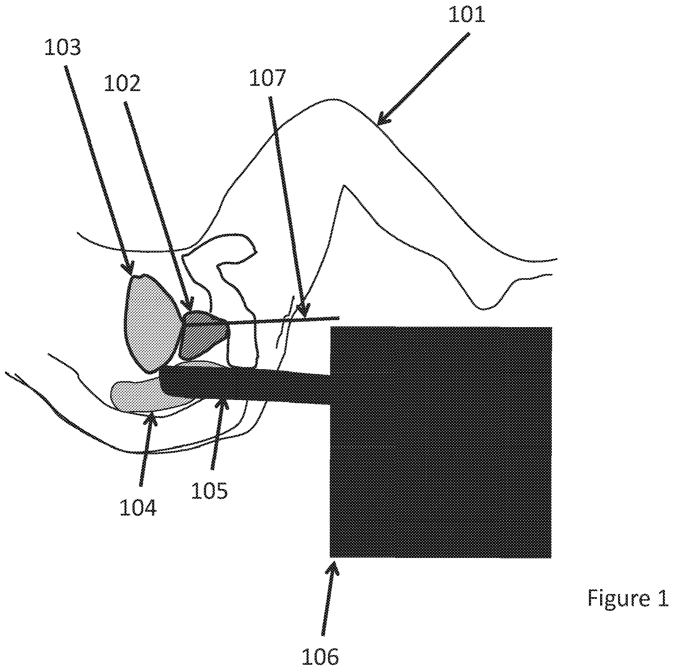

In particular, Figure 1 shows a diagram illustrating a prostate brachytherapy procedure in which the patient 101 can be positioned in lithotomy. The urinary bladder 103 is superior and adjacent to the prostate 102. The US probe 105 can be part of the US machine 106 that can be inserted into the patient's rectum 104. Brachytherapy needle 107 can be inserted through the perineum into the prostate.

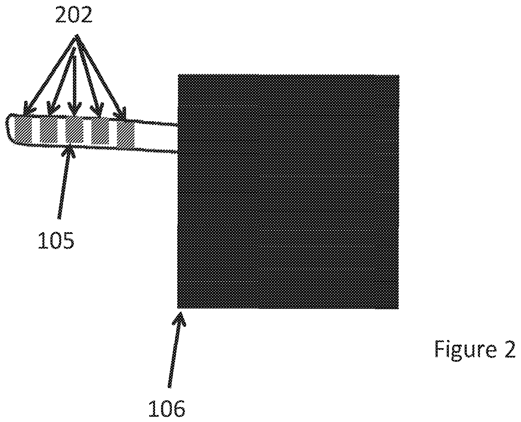

A standard US probe can detect one section of the prostate at a time; either the axial section or the sagittal section thereof. A standard US probe can include a sagittal probe and a axial probe. As shown in Figure 2, in order to acquire axial sections of the prostate (e.g., to image a larger area of the prostate), US probe 105 can have several US emitters and detectors, which can be aligned with respect to one another (e.g., 202), so that sequential imaging of all the slices can be performed without moving the probe. Because the sound velocity can be high, for example, about 300 m/sec, acquiring images of all the prostate slices can be possible in a fraction of a second, thereby providing the physician an impression of real-time imaging of all the prostate sections.

Another exemplary option can be to provide a standard US probe with a axial detector, and have this detector move in and out, such that sequential acquisition of US images of the whole prostate can be performed.

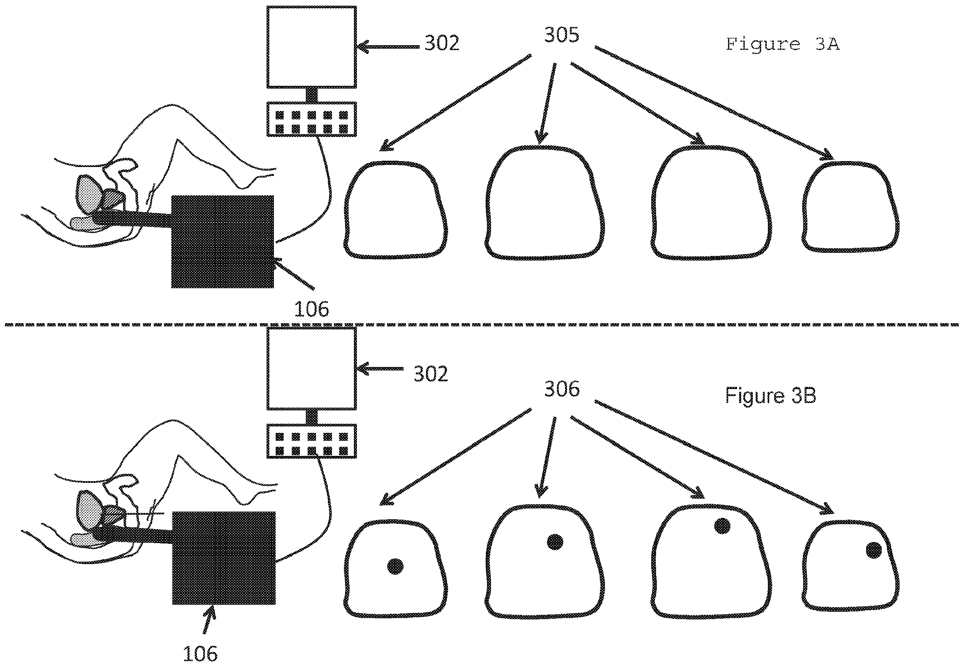

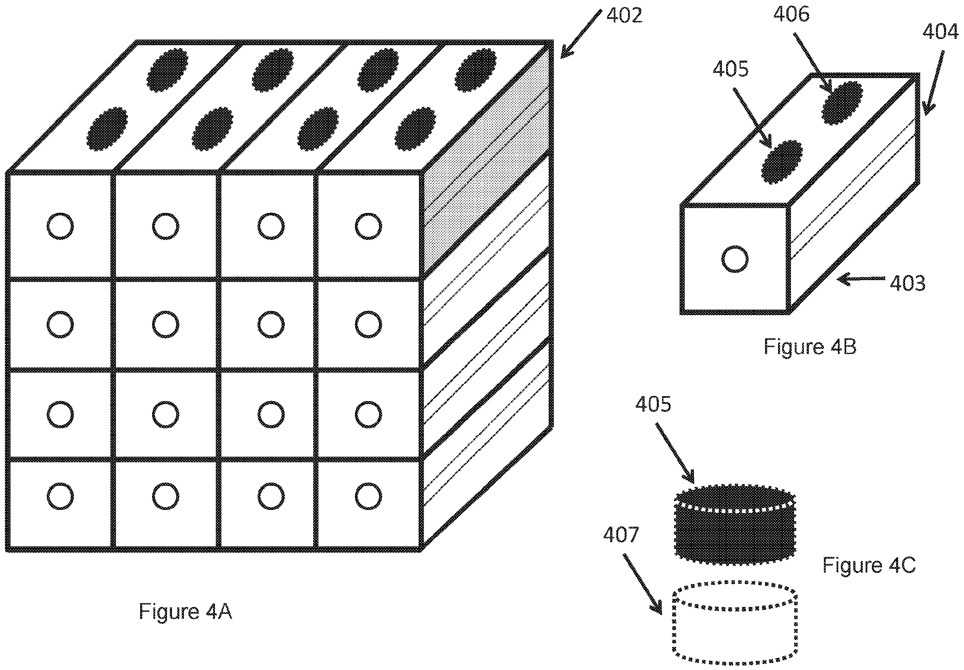

Figure 3A shows an exemplary diagram of an exemplary prostate brachytherapy procedure before brachytherapy needle insertion according to an exemplary embodiment of the present disclosure. Figure 3B shows an exemplary diagram of an exemplary prostate brachytherapy procedure after the brachytherapy needle insertion according to an exemplary embodiment of the present disclosure. For example, axial sections of the whole prostate can be acquired by the US machine 106, and displayed by the computer system 302. A set of axial images covering the whole prostate can be obtained before needle insertion 305, and after each needle insertion 306. The computer system 302 can then compare each of the corresponding US sections obtained before and after the needle insertion, and determine the location of the inserted needle in the prostate (e.g., using a digital subtraction procedure). The name of the needle can be supplied manually to the computer system, and/or can be supplied by an electronic template grid 402. Figures 4-6 show an electronic template grid 402. Figures 4A and 4B shows diagrams of an exemplary template *(see, e.g., Figure 4A)*, a unit of the exemplary template *(see, e.g., Figure 4B)* with one needle path 403, and distal LED 405 and proximal LED 406 and two light detectors 407 and 408. Figure 4C shows a pair of the LEDs 405 and the detector 407.

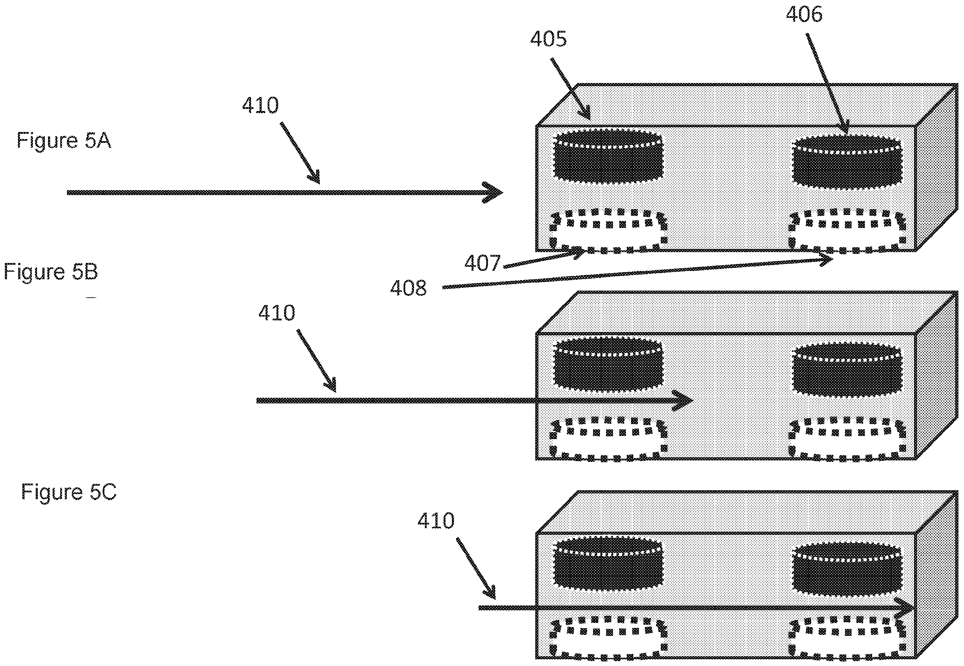

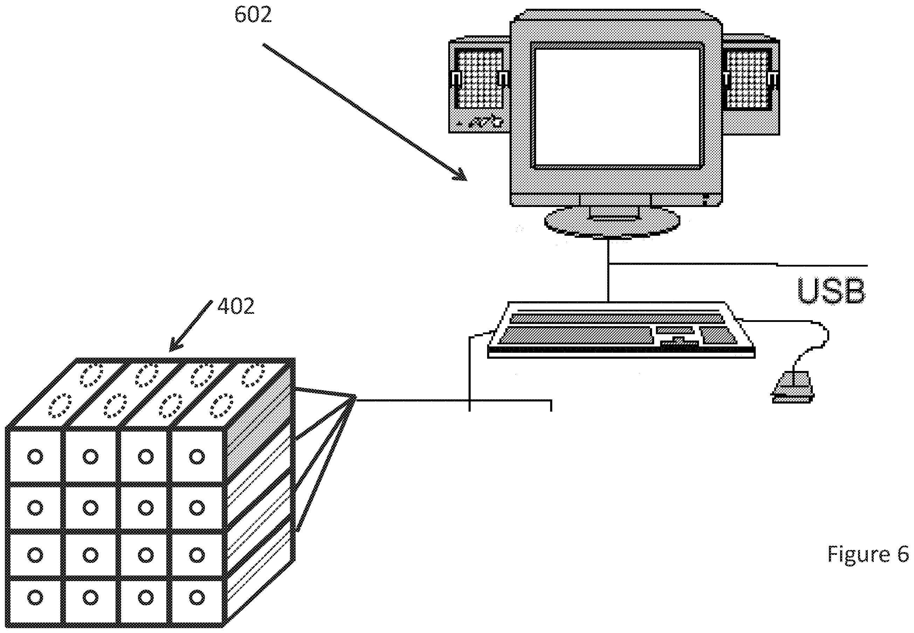

Figures 5A-5C show a diagram of a procedure in which a needle 410 is inserted in the needle hole 404, according to an exemplary embodiment of the present disclosure. For example, before the insertion of the needle *(see, e.g., Figure 5A)*, the proximal and distal light detectors 408 and 407 can detect the light emitted by the proximal and distal LEDs 406 and 405. The insertion of the needle 410 in the hole can result in hiding the light emitted from the distal LED 405 from the distal light detector 407, initially, and then resulting in hiding the light from the second LED 406 from being received by the second light detector 408. Removing the needle 410 can result in the opposite event. Figure 6 shows an exemplary diagram of the exemplary template connected to a computer system 602, which can result in an automatic assigning of names to each needle according to the coordinates of the hole that was used, and an automatic triggering of the US to obtain a set of ultrasound images after the insertion of each needle.

*Exemplary Brachytherapy Needle Insertion Procedure*

The exemplary system, method and computer-accessible medium, according to an exemplary embodiment of the present disclosure, can be used to provide a plan for the location of each needle (e.g., which can be used to implant a radioactive seed), and can update the plan (e.g., in real time) after each needle is inserted. For example, the exemplary system, method and computer-accessible medium, according to an exemplary embodiment of the present disclosure, can generate a computerized plan for the location of the deposition of the radioactive seeds, which can include the total number of seeds to be implanted (e.g., which can correspond to the number of needles insertions needed). The plan can be based on the patient's anatomy and the tumor size. After the computerized plan has been generated, the patient can be prepared for seed implantation, including having an US probe inserted into the patient's rectum, as provided in the exemplary procedure illustrated in, *e.g.,* Figure 1. After the US probe has been inserted into the patient, one or more initial US images can be generated as a baseline showing no needles having been inserted into the patient. These baseline images can be used in an exemplary digital subtraction procedure to determine the particular needle location. The electronic template grid 402 *(see, e.g.* Figure 4A)* can then be placed on or adjacent to the needle insertion site on the patient.

After the electronic template grid 402 is placed on or adjacent to the needle insertion site, a first needle can be inserted into the patient based on the needle insertion plan. The needle can be inserted into a needle hole 404 in template grid 402. Prior to needle insertion into hole 404 LED detectors 407 and 408 can detect light from their respective LED emitter (e.g., LED emitters 405 and 406, respectively). As the needle is inserted into needle hole 404, first, light from LED 405 will be obstructed, and LED detector 407 will no longer detect the light emitted from LED emitter 405. As the needle is further inserted into hole 404, light from LED 406 will be obstructed, and LED detector 408 will no longer detect the light emitted from LED emitter 406. The needle can then be further inserted through the electronic template grid 402, into the body of the patient.

The exemplary system, method and computer-accessible medium, according to an exemplary embodiment of the present disclosure, can determine that there has been a needle insertion, based on the absence of the detection of light (which can be determined by a light sensor), and associate the needle insertion with a particular point on the grid. The exemplary system, method and computer-accessible medium, can then generate one or more additional US images, having the inserted needle therein, and using the baseline US images, the exemplary system, method and computer-accessible medium, can determine the location of the needle (e.g., using an exemplary digital subtraction procedure), and register a name to the needle (e.g., based on the placement in the template grid 402).

After the initial needle insertion and the determination of the position of the needle, the exemplary system, method and computer-accessible medium, according to an exemplary embodiment of the present disclosure, can electronically compare the actual needle insertion to the planned location of needle insertion, and the exemplary system, method and computer-accessible medium can update or modify the plan, as needed, based on the needle insertion. For example, if the actual needle location is sufficiently close to the planned radioactive seed location, such that the original plan can still be the preferred plan, then no modification of the plan is needed. Alternatively, the plan may need to be updated (e.g., the location of the next needle) in order to achieve a similar treatment plan.

Based on the updated, or non-updated, treatment plan, a second needle can be inserted into the template grid and into the body of the patient. The exemplary system, method and computer-accessible medium, according to an exemplary embodiment of the present disclosure, can then electronically determine the location of the second needle (e.g., using the exemplary digital subtraction procedure based on the images obtained after the insertion of the first needle), and then name the needle based on the location of the needle insertion into template grid 402. The exemplary system, method and computer-accessible medium, according to an exemplary embodiment of the present disclosure, can then update or modify the treatment plan based on the location of the second needle. Further needle insertions, can be performed, and the exemplary system, method and computer-accessible medium, can electronically modify or update the treatment plan (e.g., including determining new needle insertion locations) after each needle insertion based on the determined actual needle location. Additionally, one or more needles can be removed based on the further locations of the needles. The exemplary brachytherapy needle insertion procedure can be complete after the exemplary system, method and computer-accessible medium can determine that the actual needle location is sufficient to perform the radiation therapy treatment plan (e.g., based on the seed location corresponding to each needle location).

*Exemplary Determination of Seed Location*

The exemplary system, method computer-accessible medium, and apparatus according to an exemplary embodiment of the present disclosure, can also utilize multi-modal imaging (e.g., ultrasound, cone beam CT, diagnostic CT, magnetic resonance imaging and/or positron emission tomography- PET-CT scanning) to capture seed coordinates in a precise way, as part of the procedure, and while the patient is in the treatment position. In general, prostate implants can be performed under TRUS guidance. TRUS can be advantageous because the patient anatomy can be easily and reliably discerned. Post implant evaluations, in contrast, are typically conducted under CT guidance. The reason for this is that CT simulators can be widely available in radiation oncology departments. CT can also provide a superior visualization of the implanted seeds. However, CT can be a less-than-ideal imaging modality for the prostate and other clinically relevant organs. Further, this can create an ambiguous situation where different, not-comparable, imaging modalities can be used interchangeably. In contrast, the exemplary system, method computer-accessible medium and apparatus can combine the superior anatomic delineation using TRUS, with intraoperative CT, or Cone Beam CT ("CBCT"), for the visualization of the seeds. The combined information can be fed into the intraoperative planning system for evaluation, and any changes can be immediately implemented during the seed implantation procedure while the patient remains under anesthesia.

*Exemplary Procedure*

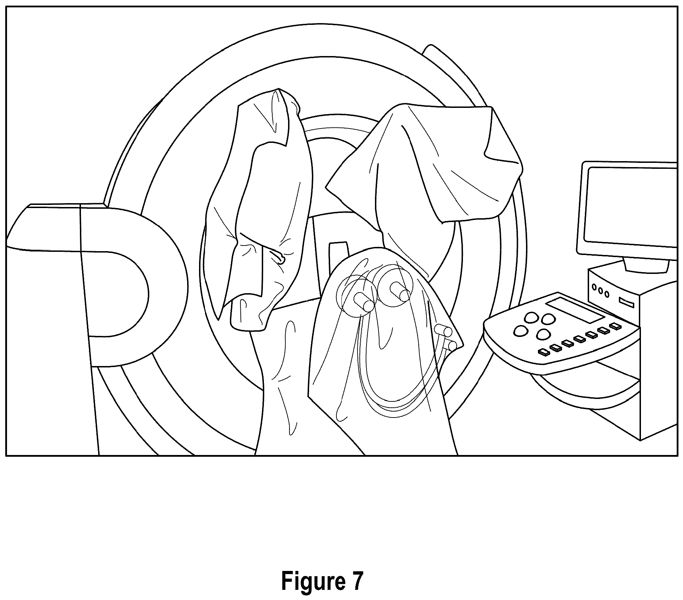

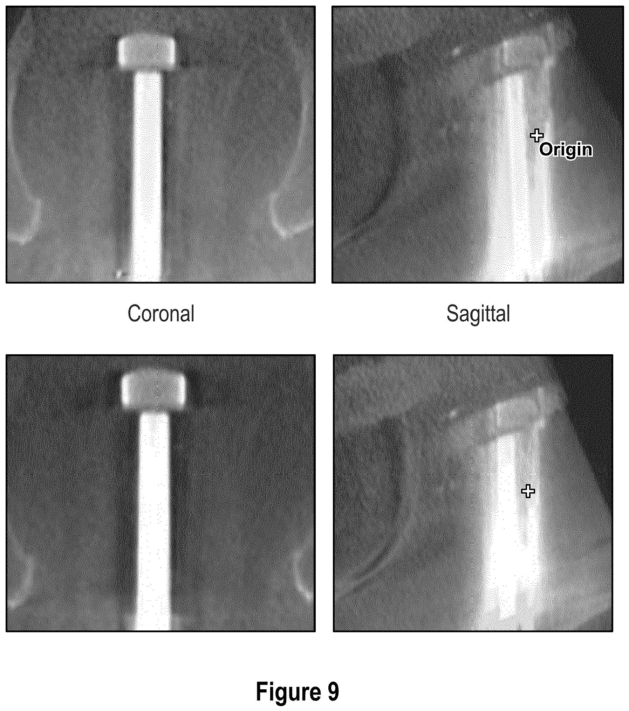

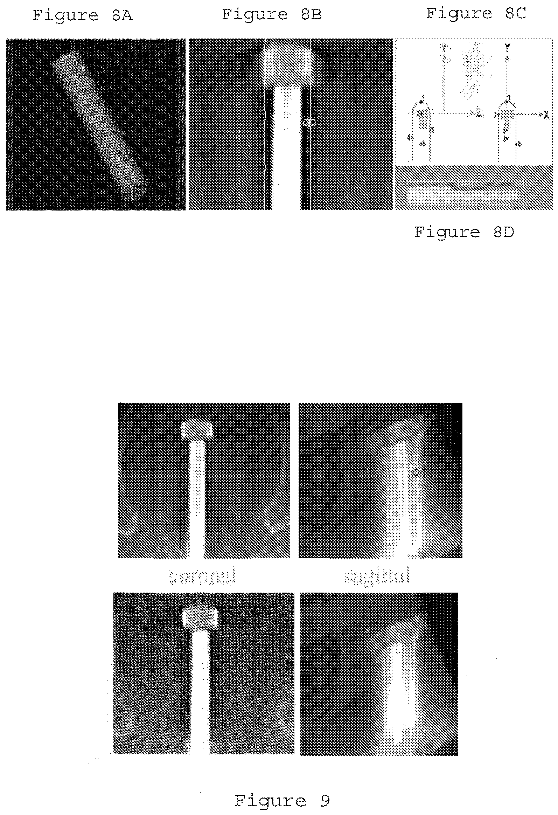

The patient, under anesthesia, can undergo a prostate implant: (i) TRUS imaging, (ii) planning and (iii) deposition of some or all of the planned seeds. As part of this exemplary procedure, the patient can be placed in an extended lithotomy position, such that an ultrasound probe can be introduced trans-rectally, and, at the same time, a mobile CT unit can be used for imaging the same area. *(See, e.g.,* Figure 7). After some, or all, of the planned seeds have been implanted, and with the patient still in the implant posture, a CBCT/CT image can be acquired, along with an image from the TRUS probe already inserted into the patient. An additional set of ultrasound images can be acquired with the patient in the same posture. The transformation between the CT space and the TRUS space can be calculated. This can be a rigid Cartesian transformation in X, Y, and Z and angles a, b, and c about axes X, Y, and Z. The exemplary procedure can be performed by matching objects that have known coordinates in any two image spaces. For example, a template CT image set that includes a three dimensional reconstruction of the ultrasound probe, and for which the coordinated of the ultrasound probe ultrasound space are implicitly known (*see, e.g.,* Figures 8A-8D), can be registered to a newly acquired CT image set of the patient that can also include a reconstruction of the same ultrasound probe. *(See, e.g.,* Figure 9). This registration, achieved using image intensity profile matching, can yield the rigid Cartesian transformation matrix that can be used to transform the seed coordinates and anatomical contours from the US space to the CT space and vice versa. For added image registration robustness, fiducial makers can be added to the US probe. For example, six such markers can be added in a non-coplanar configuration. (*See, e.g.,* Figure 8C). Reads of planned seeds positions, and anatomical data from the original TRUS plan can be projected on to the new CT images. The planned seed locations can be used as an initial approximation for detection (e.g., location) of the actual "deposited seeds" in the CT image set. The procedure can have strict criteria for the detection of seeds. The exemplary system, method and computer-accessible medium, according to an exemplary embodiment of the present disclosure, can search the CT volume for contiguous pixels of high Hounsfield numbers, such that the volume of each set of contiguous pixels can equal the volume of a seed. Objects that have the density of seeds, but can be incompatible with the known volume of a seed can be marked as "likely seeds," and listed for the user to decide whether to re-label them as deposited seeds or not. This can be beneficial, and can save time, as there can often be fiducial markers, calcifications, or other dense objects that previously known procedures can mistake for seeds. Additionally, two seeds can be close together, and not be readily recognized as seeds. The likelihood of high density pixels being a seed can be ranked based on the intensity of the pixels, the volume of the contiguous pixels, and the distance of the pixels from a planned seed.

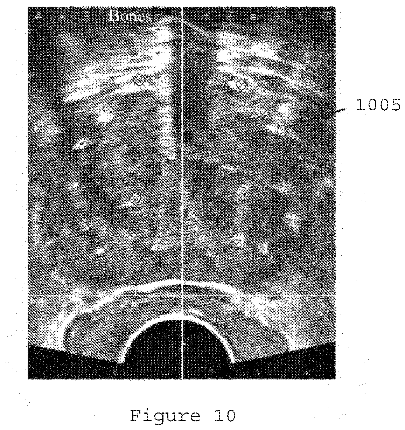

As shown in an exemplary image in Figure 10, after all of the seeds 1005 have been marked, the newly detected seeds, along with the anatomical data from the original planned locations, can be merged onto the new TRUS images. This can be performed based on the calculated transformation above.

The contours can be adjusted to account for changes in anatomy, and the new dosimetry can be available immediately for evaluation. If the implant does not need any changes, the process can stop here, and the dose calculation just performed can serve as the post implant evaluation. If changes can be needed, as there can be recognition based upon this aforementioned intraoperative evaluation of suboptimal dose delivery to the target, additional seeds can be planned and the location of these seeds can be optimized given the dose from previously implanted seeds. If additional seeds are needed, the exemplary process of intraoperative evaluation can be repeated, where the most recent ultrasound-based evaluation/plan can serve as the original plan for the new cycle. The whole process can take approximately 10-15 minutes in an operating room. This can be comparable to the time it takes to generate the original plan. This exemplary procedure can be repeated several times until the implant meets all expectations, and is beneficial as it can be performed during the initial seed implantation procedure.

Thus, the exemplary system, method and computer-accessible medium can be used for (i) intraoperative CT seed localization, (ii) automated seeds localization process and (iii) probe template registration to establish transformation between TRUS and CBCT. Prior knowledge of seed location, or proposed seed location, can be utilized to increase the speed and accuracy of the exemplary system, method and computer-accessible medium.

The exemplary system, method and computer-accessible medium, according to an exemplary embodiment of the present disclosure, can also be used to identify and address user errors, which can often be encountered during a seed implantation procedure. These errors can be related to misidentification of needles and seed counts. In order to overcome these problems the exemplary system, method, computer-accessible medium and apparatus can detect, electronically (e.g., using a template of seed location), the passage of a metallic object *(e.g.* a needle or a seed) through the body, and can indicate which location *(e.g.,* in the template) is being used, and how many seeds are being deposited through that template location. During the exemplary template generation, the exemplary system, method, computer-accessible medium, and apparatus, according to an exemplary embodiment of the present disclosure, can interface with a treatment planning system/process to automatically guide a user *(e.g.,* a physician) through the implantation process based on the planned seed implantation locations.

Thus, the exemplary system, method, computer-accessible medium, and apparatus, according to an exemplary embodiment of the present disclosure, can enhance the performance of seed implants by providing a consistent procedure for achieving high quality implants, which can reduce the likelihood of inferior dose delivery to the cancer, and can avoid medical events which, as a result of operator error or misplaced seed deposition, can lead to cancer recurrence.

**Exemplary Radiation Therapy**

*Exemplary Clinical Overview*

Preferably, radiation treatments deliver a curative dose to the tumor and completely spare surrounding healthy tissue. In practice, however, delivering the dose needed for local tumor control can result in unacceptable normal tissue complications, in effect placing an upper limit on the dose deliverable using external beam radiation therapy ("EBRT"). This can be of a particular relevance for locally recurrent tumors, some of which may have been treated previously, and pediatric tumors, where normal tissue toxicity needs special consideration. Various treatment techniques have been proposed to overcome this limitation, including external beam ("EB") intensity-modulated radiation therapy ("IMRT") and brachytherapy.

Briefly, IORT refers to a single fraction treatment delivered to a surgically exposed target area. Two competing approaches to IORT are currently in practice: the first is linac based using electron beams, the second employs a HDR 192Ir afterloader. The brachytherapy-based intraoperative approach can be of particular interest because it can be best suited to maximize the therapeutic ratio and deliver doses higher than any other treatment modality. The treatment can be performed at the time of surgery, when the target area *(e.g.,* the tumor bed) can be exposed, and the applicator can be placed directly over the target. Organs at risk can be retracted and shielded as necessary. This exemplary procedure can be free of any accessibility limitations; the applicator can be used in virtually any anatomic location. This can be important in the treatment of colorectal malignancies, where the tumor bed can often be inaccessible to the cones of a linac-based system *(See, e.g.,* Reference 19).

The flexible applicator used for HDR-IORT can easily conform to the target area, ensuring uniform dose delivery throughout the target (e.g., avoiding cold spots and hot spots often encountered when using the electron beam approach as a result of angle of beam incidence and field matching). Beyond clinical and technical considerations, IORT can offer the convenience and cost effectiveness of accelerated-course radiotherapy used more recently for the treatment of breast cancer.

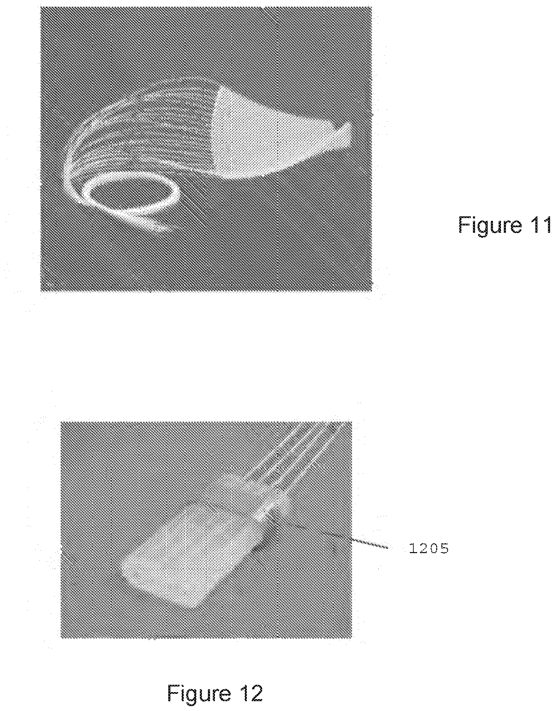

The standard HAM applicator *(see, e.g.,* Figure 11) can include standard-length (e.g., about 130 cm) catheters embedded at about 1 cm spacing in about 8 mm thick silastic rubber, with about 5 mm from the center of catheters to the front of the applicator and about 3 mm to the back. The asymmetry, reducing the overall thickness of the applicator, was chosen for added flexibility. The number of catheters can vary from 2 to 24, and the length of the silastic can be about 22 cm, resulting in a treatment area up to about 23 cm in width and about 20 cm in length. The prescription point can usually be about 0.5 cm from the surface of the applicator *(e.g.,* or about 1 cm from the plane of the catheters).

An exemplary modified HAM applicator for use in breast-IORT is shown in Figure 12. For example, the silastic rubber can be molded symmetrically with at least about 1 cm from the surface to any of the catheters. This applicator can be fitted with a tungsten shield to protect the skin at the incision. Prescriptions using this applicator can usually be about 1 cm from the surface of the applicator (e.g., about 2 cm from the source plane).

### Exemplary Cost of IORT Brachytherapy

On the average, currently, the applicator cost can be about $100.00/channel. Source exchange and periodic maintenance (e.g., assuming quarterly source exchanges) can be approximately $50,000.00/year. With an average treatment width of five channels, and one treatment per week, the cost per treatment, excluding initial investments, can be approximately $1,500.00.

*Exemplary Treatment Planning*

### Exemplary Dose Specification

In an operating room ("OR"), this can arguably be one of a few instances in which a medical physicist will be working upon oral instructions rather than on written directive; however, standard OR procedures should be followed: details of the prescription should be clearly repeated by the physicist and confirmed by the physician. An IORT prescription typically specifies the width *(e.g.,* or number of channels), the length *(e.g.,* or number of positions if the source stepping distance can be known), the dose needed, and the prescription point. Typically, a homogenous dose distribution can be desired throughout the treatment area, with doses in the range of about 15 to about 20 Gy. Lower doses (e.g., about 10 to about 15 Gy) can usually be used for pediatric cases, and when IORT can be used as a boost in conjunction with EBRT treatment. Occasionally, nonrectangular target areas and critical organs can be specified. The orientation of the applicator must be established to implement such dose prescriptions correctly.

### Exemplary Treatment-Planning Approaches

Most IORT treatment plans can assume a fixed applicator geometry, and can be generated from pre-calculated plans or template-based plans. These can essentially be fixed treatment geometries that can be stored in, or automatically generated by, the exemplary treatment-planning system. These treatment-planning schemes can be attractive mainly for their simplicity, reliability and fast output *(see, e.g.,* Reference 16).

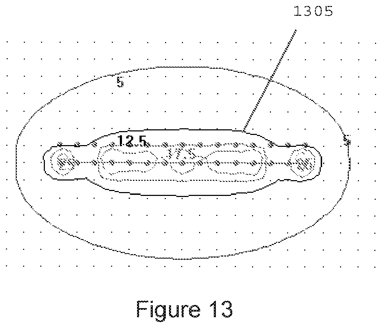

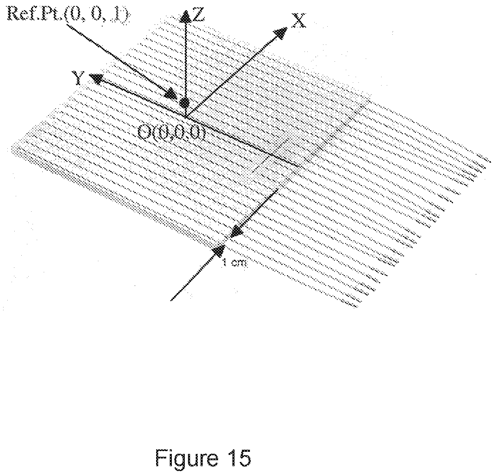

In some circumstances, custom plans can be needed to accommodate regions of dose escalation or dose sparing. Figure 13 shows a diagram of an exemplary plan with a dose escalation region in the middle of the treated area. Standard IORT plans can be symmetric by nature. However, this may not be the case for custom treatment plans. In these cases, the orientation of the applicator with respect to the patient *(e.g.,* channel 1-Med; applicator tip-Post) should be examined; channel numbering tags can be mounted on the catheters for this purpose. *(See, e.g.,* Figures 11 and 15).

While efficient dose sparing methods can utilize a combination of retraction and shielding of the organs at risk, in some cases, anatomic/geometric constraints require that the dose be tailored using treatment plan optimization. Breast IORT, for example, aims at delivering about 20 Gy to a surface about 1 cm away from the applicator while sparing the skin (e.g., ideally the skin dose should not exceed about 10 Gy). In this type of geometry, the skin can be at the applicator surface, and very close to the treatment volume. It can usually be possible to achieve a skin dose of about 15 Gy by means of dose optimization, and further reduce the skin dose to about 10 Gy using shielding. *(See, e.g.,* Figure 12, element 1205).

### Exemplary Computer-Assisted Plan Verification

Plan verification, unlike treatment planning, may not be implemented in commercial software packages. Several procedures have been suggested *(see, e.g.,* References 18, 24, 27, 28 and 29), most of which can address only single-catheter treatments. HDR brachytherapy plans can be complex in nature; they involve many source-stopping positions with widely varying dwell times and the time allotted for planning and verification can be limited.

Consequently, it can be impractical to manually review the plan for errors. To address this problem, various implementations of computerized QA using spreadsheet calculations can also be used. Typically, the physicist will type dwell times into a spreadsheet form, and calculate the dose at one or more points of interest. However, manual data entry can slow the QA process, and can make this susceptible to errors, especially if used for large implants. As a way to independently verify a plan a computer program can be used to assist in this task *(See, e.g.,* Reference 17). Below is a description of an exemplary system, method and computer-accessible medium that can be used for HDR planning. The exemplary discussion below can pertain to any procedure (e.g., whether or not invasive, or under anesthesia) for which a patient can be on the treatment table, waiting for the plan to be completed and the treatment to commence. This definition includes cylinders for vaginal treatment, ring and tandem for cervical cancer, and endobronchial treatments of the lung. The QA of HDR treatment planning in general has been discussed extensively in the report of AAPM TG-59 *(See, e.g.,* Reference 21). This program can be an efficient tool assisting the physicist in implementing AAPM recommendations.

Once a treatment plan is completed, the treatment-planning system can generate a data file, which can contain all the parameters necessary for treatment. This information, which can be transferred to the afterloader console for treatment delivery, can serve as the main data source for the independent verification program. Use of this file for secondary-dose calculations can offer several obvious advantages. It can provide an orderly way to: (i) read treatment parameters into the dose verification program, (ii) confirm that the treatment data file corresponds to the actual treatment plan, (iii) ensure that the data file can be intact and (iv) use actual treatment parameters to both verify treatment geometry and perform a secondary dose calculation.

The secondary verification can include two procedures: (i) determination/validation of treatment geometry, and (ii) dose calculation based on that geometry. Once these two exemplary procedures can be completed, the independent reviewer can verify that the treatment file conforms to the desired treatment parameters. User input can be minimal.

### Exemplary Determination of Implant Geometry

An exemplary task in the determination of implant geometry can be the correct reconstruction of dwell positions ("DPs"). Each DP can be defined by a nominal dwell time, and a set of coordinates in the patient's coordinate space *(e.g.,* X, Y, Z). For fixed geometry implants, DP coordinates can be implicitly defined by the configuration of active channels. In general, for other implant geometries such as template-based implants, DP coordinates can be approximated using explicit user input.

Applicator geometry for most or all procedures described herein can be fixed, and can be encoded in the software. The computer program can reconfigure the computer to automatically detect the type of applicator being used, virtually eliminating user input. For example, the number of active channels and the channel index can indicate which applicator can be used. Because for most applicators the location of the prescription point can also be fixed, the complete implant geometry can be automatically reconstructed. In the case of IORT, e.g., the active treatment area can be defined by the length L *(e.g.,* Y-axis) and width W (e.g., X-axis), with the origin at the center of the implant. *(See, e.g.,* Figure 15). Assuming a flat applicator *(e.g.,* Z = 0 for all DPs) with N channels and M positions per channel, the coordinates of DP(n,m) can be given by:

$$(X, Y)_{n,m} = \left[\left(\frac{N-1}{2} - (n-1)\right) \times ChSp, \left(\frac{M-1}{2} - (m-1)\right) \times Step_n - Shift_n\right] \qquad (1.1)$$

where "Shift" can be the distance the first dwell position can be shifted from the tip of the applicator, "Step" can be the distance between dwell point positions, and "ChSp" can be the spacing between channels, which in the case of the HAM applicator can be fixed and can equal 1 cm. *(See, e.g.,* Figure 11). In the case of a single channel application, where N=1, Xn,m can reduce to X1,m = 0. It should be noted that in the specific case of the HAM applicator, each "atlas" treatment plan can use a fixed length *(e.g.,* M can be constant) for all channels. This, however, need not be the case, because the computer program can reconfigure the computer to read all 40 dwell times for each of the 24 channels, whether or not they are used. The detection of the applicator type, and the calculation of the dose at the reference point(s) can be performed as follows.

**Exemplary Single Channel Applications:** (e.g., channel no. < = 19) can indicate a vaginal applicator; (e.g., channel no. > = 20) can indicate a bronchial/esophageal applicator. The GammaMed afterloader can automatically test the length of channels 1 to 19 *(e.g.,* of 24 channels) to ensure that the first dwell position can correspond with the end of the catheter/tube. Unlike vaginal applicators, endoesophageal and endobronchial applicators can be subject to distortion and variations in length, and can typically be attached to channel no. 24 of the afterloader. The dose reference point for a vaginal procedure can be set at Pref = (0.0, 0.0, d/2+0.5) cm, where d can be the cylinder diameter. Because d can be variable, the distance of Pref from the source channel can be entered manually. For bronchial or esophageal procedures, Pref = (0.0, 0.0, 1.0) cm.

Two active channels can indicate a GYN ring and tandem applicator. Here, the user may need to enter the length of the tandem. With the tandem orthogonal to the plane of the ring, and the channel in the ring section of the applicator also fixed, the applicator geometry can be defined. In some cases (e.g., applicable only when the tandem can be made of two sections), the angle of the sleeve may not be perpendicular to the plane of the ring; then user input can be needed. The reference points can be defined as points Aright and Aleft and can be fixed relative to the applicator hardware. The outer diameter of the ring *(e.g.,* of the caps) can also be entered, to calculate the dose to the vaginal mucosa. Uterine and cervix points can also be fixed with respect to the applicator, and can automatically be calculated.

Three or more active channels can indicate a HAM applicator. An exemplary assumption can be that the applicator is likely flat. This can be an appropriate approximation for most procedures. For HAM-IORT, e.g., the dose reference point can be set at Pref = (0.0, 0.0, 1.0) cm *(e.g.,* 1.0 cm from the source plane, or 0.5 cm from the surface of the applicator) and for breast IORT at Pref = (0.0, 0.0, 2.0) cm. In the case of a convex curved application (e.g., rectal IORT), the dose calculation can predict an overdose of about 5% to about 10%. While this can still be within regulatory requirements, the exemplary treatment planning "Atlas" and dose verification program can both accommodate the curved geometry.

### *Exemplary Dose Calculation*

Dose-to-point calculations can include a summation of dose contributions from all dwell positions, using a two-dimensional dose table, F(r,σ), for the 192Ir source, and the fifth-order Meisberger polynomial, M(r) *(See, e.g.,* Reference 22). Both the dose table and polynomial can be hard coded in the exemplary system, method and computer-accessible medium, making it self-contained. Dose calculations can typically be within 1% to 2% of the planned dose.

By the end of plan verification, the total treatment time can also be known. The total treatment time (e.g., including dummy tests) can be communicated to the anesthesiologist, to ensure proper medication of the patient. Also, at this time, surgeons, the radiation oncologist and the OR staff can be ready to start treatment. For example the last step in the plan verification process can be approval (e.g., oral) of the plan by the physician. It can be important to "pause" and review the plan with the physician; this can be the last chance to catch any miscommunications or other errors in an otherwise busy and time-pressured environment.

### *Exemplary Classical Dosimetric Systems*

To complement the plan verification discussed herein, for example, a classical Manchester system can be used. The original Manchester tables were designed to give the total amount of radium *(e.g.,* in mg) times application time *(e.g.,* 1 in hours) needed to deliver 1000 R for various implants, given the implant's volume or area, and elongation. *(See, e.g.,* Reference 14). This exemplary quantity, M, differs only by a constant from total reference air kerma ("TRAK") expressed in gray. *(See, e.g.,* Reference 20). Because mg·h can still be most commonly used, M can be expressed as the total mg·h that can produce about 10 Gy in water. Using the ratio of exposure rate constants for radium and iridium, and adjusting for units, the total dwell time for a nominal source activity of 370 GBq, expressed in s/Gy, can be interpolated from the Manchester table, M (expressed in mg·h), as follows:

$$ T = 0.0659 M \left( A \left( \frac{0.5}{h} \right)^2, 0.5 \right) \left( \frac{h}{0.5} \right)^2 \exp\left( 0.05[E-1]^{\frac{3}{4}} \right), \qquad (1.2) $$

where h can be the distance (e.g., in cm) from the plane of source to the treatment plane, A can be the treatment area *(e.g.,* in cm$^2$), and E can be the elongation of A. The original Manchester tables were written for a treatment distance of about 0.5 cm from the plane of the source. The equation above reflects the use of the table at other treatment distances as well. Predictions of the total nominal dwell time using this method can usually be within about 5% of the planned time.

### *Exemplary QA Considerations*

Exemplary QA procedures should follow standard recommendations *(See, e.g.,* Reference 26). However, it can be beneficial to remember that IORT can be a single-fraction treatment. Proper QA of the hardware, software, and each treatment plan can be beneficial for a successful IORT program.

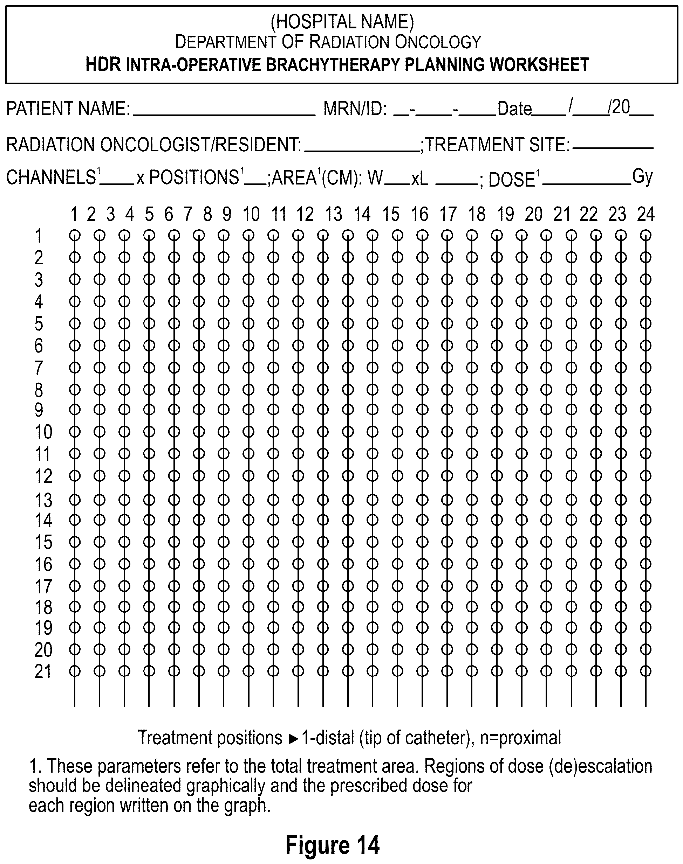

The exemplary applicator can be or include a single-use device that can require in-house sterilization. Each applicator can undergo QA prior to sterilization. For example, the QA procedure can include checks of catheter integrity and lengths, catheter labeling, and overall applicator integrity. If source transfer tubes can be used, the QA can include them, and they should be sterilized as well. It can be important to examine the applicator after the procedure can be complete and to establish its integrity; if lead shields or source transfer guides were used, it can be important to ensure that all can be intact and accounted for. Initial QA at the time of the IORT program is being set up should be performed to ensure the applicator and source transfer tubes are not damaged during the sterilization process. The operation room can be a high-pressure environment. It can be recommended that standardized forms and treatment protocols be used whenever possible. An example of a treatment setup form is shown in Figure 14.

**Exemplary Independent Dose-To-Point Calculation Program For The Verification Of High-Dose-Rate Brachytherapy Treatment Planning**

***Exemplary Methods And Materials***

Currently, two GammaMed 12i HDR remote afterloading machines have been used, and located in a dedicated operating room suite. The exemplary system, method and computer-accessible medium according to an exemplary embodiment of the present disclosure can operate with the GammaMed data files, and can follow the design philosophy of these machines. Specifically, because the afterloader always sends the source to the distal end of each channel, and only then performs the treatment while retracting the source, the first source stopping point ("SSP") can be at the distal end of each channel. Each afterloader can independently store source calibration data (e.g., initial activity and date of calibration); thus differentiation between afterloaders can, and should, be done at the time of treatment delivery. Therefore, planning can be independent of the afterloader used. Most or all dosimetric calculations can be performed for a nominal 37·GBq (10 Ci) source activity. Treatment planning systems interface with after-loading machines via network, or manually, via diskette, magnetically encoded card, memory stick or another data storage device, and format of the data files differ between manufacturers. The exemplary system, method and computer-accessible medium, according to an exemplary embodiment of the present disclosure, can be easily adaptable to other HDR units, file formats and treatment practices.

For most fixed-geometry procedures, treatment planning can include the use of an "atlas" *(see, e.g.,* Reference 11) of precalculated plans. In-house CT and ultrasound-based treatment planning software can be used for prostate HDR, and GammaMeds Abacus™ software can be used for other procedures. Once a treatment plan can be completed, the treatment planning system can generate a data file, which can contain all the parameters needed for treatment, which can include, for example:

1. Patient identification: medical record number, name, fraction number; and
2. Channel parameters: shift of first SSP with respect to standard end of channel, source step size, total dwell time for that channel, individual dwell times for each SSP in that channel.

This exemplary information, which can be transferred to the afterloader console for treatment delivery, can serve as the data source for the independent verification program. Use of the same file for secondary-dose calculations can offer several advantages. It can provide an orderly way to: (i) read treatment parameters into the dose calculation program, (ii) verify patient identification, (iii) confirm that the treatment data file corresponds to the actual treatment plan (especially useful when multiple fractions can be planned), (iv) ensure that the data file can be intact, and (v) use actual treatment parameters to verify treatment geometry and perform a secondary dose calculation.

The secondary verification described in this communication can include, for example: (i) determination/validation of treatment geometry, and (ii) dose calculation based on that geometry.

***Exemplary Further Determination Of Implant Geometry***

An exemplary procedure in the determination of implant geometry can be, e.g., the correct reconstruction of SSPs. Each SSP can be defined by a nominal dwell time and a set of coordinates in the patient's coordinate space (X, Y, Z). For template based implants *(see e.g.,* case 1 below) SSP coordinates can be approximated using explicit user input.

**Exemplary Case 1**: CT-Based Template Implants. For the more general case of CT or US template-based HDR plans, the implant geometry may not be easily reproduced. The exemplary system, method and computer-accessible medium, according to an exemplary embodiment of the present disclosure, can be used for a prostate HDR treatment.

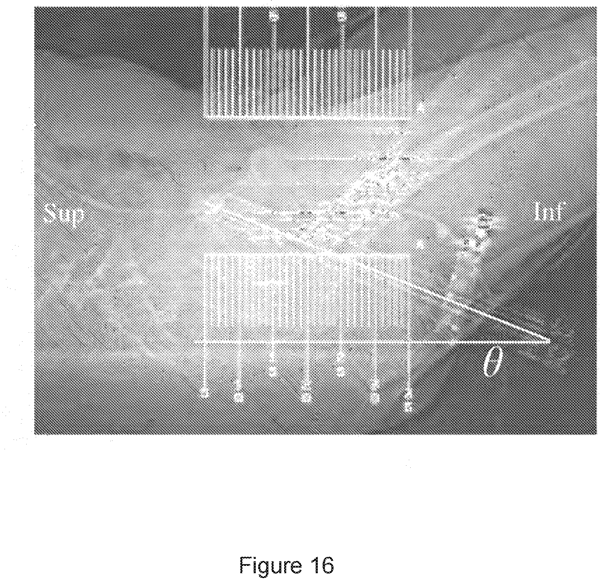

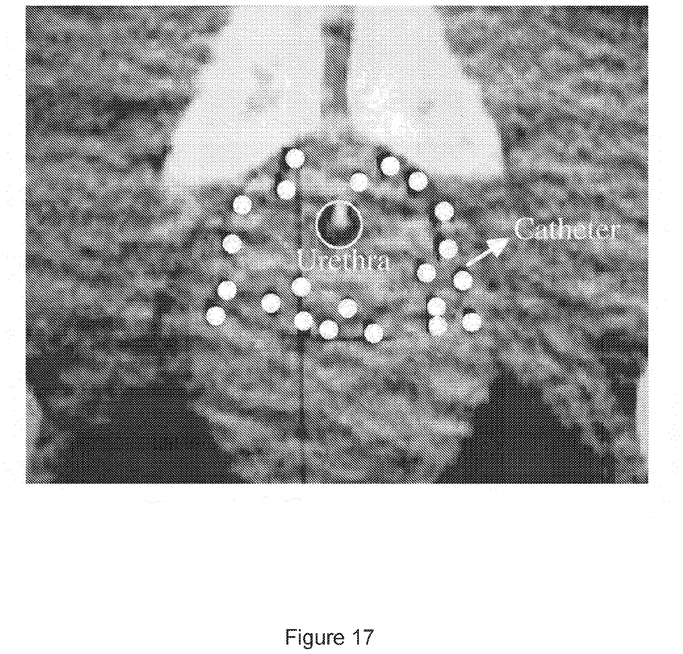

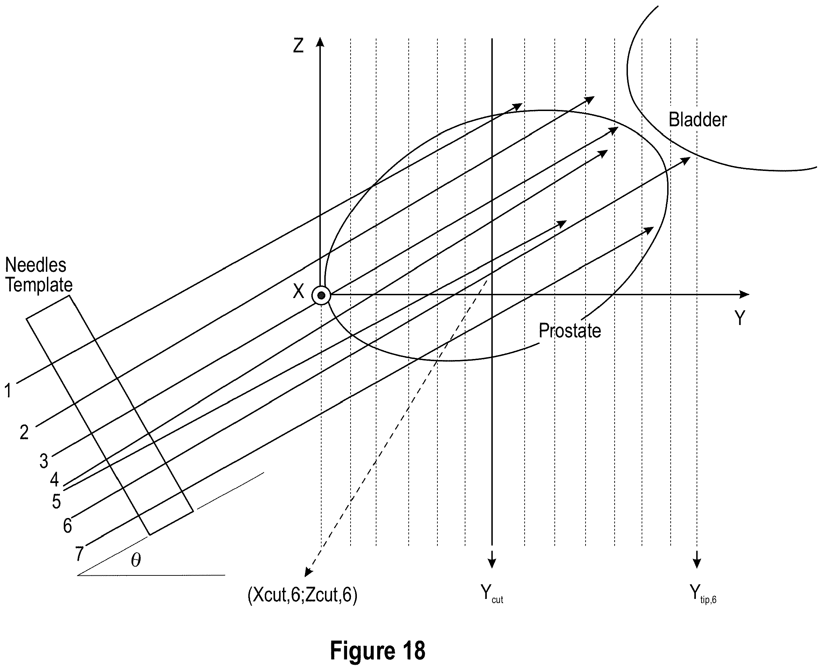

The difficulty in the reconstruction of the implant's geometry (e.g., for QA purposes or otherwise) is shown in Figures 15 and 16. In most implants, the tips of the catheters do not start at the same level on the Y-axis (e.g., superior-inferior) and are usually not orthogonal to the axial CT slices. *(See, e.g.,* Figure 16). Additionally, at the levels of the prostate, the catheters' relative positions may no longer reflect the original perineal grid coordinates. *(See, e.g.,* Figure 17). Figure 18 shows a schematic diagram of an exemplary prostate application. The coordinate origin was arbitrarily set to the CT/US origin $(X_0, Z_0)$, and the most inferior image containing a target volume $(Y_0)$. To estimate the implant geometry, the following information can be entered the Y-coordinate of each channel tip $(Y_{tip,n})$. Typically, for each catheter, the most superior CT cut still intersected by that catheter and the average angle between the catheters and the CT Y-axis can be selected. *(See, e.g.,* Figures 16 and 18). For the CT slice being checked, $(X_{cut}, Z_{cut})$ of each catheter can be entered (e.g., using the digitizer). Because catheter position can vary from one slice to the next, catheters should be digitized on each CT slice checked. *(See, e.g.,* Figure 18).

The SSP(n, m) coordinates can be estimated by:

$$X_{n,m} = X_{cut,n}$$

$$Y_{n,m} = Y_{tip,n} - \left(((m-1)x\ Step_n) + Shift_n\right)\cos\theta \quad (2)$$

$$Z_{n,m} = Z_{cut,n} + [Y_{tip,n} - Y_{cut} - (((m-1)\ x\ Step_n)$$

$$+ Shift_n)\cos\theta]\tan\theta = Z_{cut,n} + [Y_{n,m} - Y_{cut}]\tan\theta$$

Following this step, calculation points *(e.g.,* as many as needed) can be digitized $P_{ref}$ = ($X_{dig\cdot}$, $Y_{cut\cdot}$, $Z_{dig\cdot}$) and the dose to those points can be calculated.

### *Exemplary Further Dose Calculations*

Exemplary dose-to-point calculations can include a summation of dose contributions from all dwell positions, using a two-dimensional dose table, F(r, Θ), for the Iridium-192 source, and the fifth-order Meisberger polynomial M(r). *(See e.g.,* Reference 12).

An approximation of the total dwell time can be obtained in various types of implants, and the treatment planning optimizer results can resemble a Manchester loading pattern (e.g., source loading pattern can be preferentially peripheral rather than uniform). It has been suggested that optimization systems should have an option to apply clinically individualized versions of the Manchester rules. *(See e.g.,* Reference 13). An application of the Manchester tables for QA has been previously demonstrated. The original Manchester tables were designed to give the total amount of radium (e.g., in mg) times application time *(e.g.,* in hours) required to deliver 1,000 R for various implants, given the implants' volume or area, and elongation. *(See e.g.,* Reference 14). This quantity, M, can be equivalent to total reference air kerma ("TRAK") expressed in gray. *(See e.g.,* Reference 15). Because mg•h can still be most commonly used, in the equations that follow, M can be expressed as the total mg•h that produce 10 Gy in water. Thus, for an implant of volume V, and elongation E, the Manchester volume table was generated by:

$$\left(\frac{M}{mg\ h}\right) = 34.1\ \left(\frac{V}{cm^3}\right)^{2/3}\ \exp(0.07[E-1]) \quad (3)$$

The numerical constants in this expression can reflect the choice of units, as indicated. The total dwell time for an HDR iridium source can be calculated as well. Using the ratio of exposure rate constants for radium and iridium and adjusting for units, the total dwell time for a nominal source activity of 370 GBq can be T = 6.59 $10^{-2}$M [s/Gy]. The total nominal time for a volume implant can therefore be, for example:

$$\left(\frac{T}{s\ Gy^{-1}}\right) = 2.247\ \left(\frac{V}{cm^3}\right)^{2/3}\ \exp(0.07[E-1]) \quad (4)$$

Similarly, the total nominal time for a planar implant can be given by, for example:

$$\left(\frac{T}{s\ Gy^{-1}}\right) = 0.0659\ \left(\frac{M}{mg\ h}\right)_{\left[A\left(\frac{0.5}{h}\right)^2\right]}\left(\frac{h}{0.5}\right)^2 \quad (5)$$

$$x\ \exp(0.05[E-1]^{3/4}$$

where h can be the distance (e.g., cm) from the source plane to the treatment plane, A can be the area treated *(e.g.,* $cm^2$), and M can be interpolated from the Manchester tables for the value of [A X (0.5/h)2].

### *Exemplary Results*

The exemplary system, method and computer-accessible medium, according to an exemplary embodiment of the present disclosure, can be used as a fast and accurate secondary dose-calculation procedure for QA of HDR treatment planning. For general applications, such as CT-based prostate treatments, the dose calculation can be within 1-2% of the planned dose for points outside the treatment volume, and 3-5% at the periphery of the target volume. Within the target volume, as the reference points get closer to stopping positions, the dose gradient can be high, and the difference between the planned and calculated dose can be as high as 20%. For fixed geometry applications, the secondary dose calculation can be within 2% of the planned dose, and verification can take about 1 minute to complete. For general CT-based treatment plans, the verification can take approximately 5 minutes. In addition, The Paterson-Parker tables can be easily implemented to check total dwell time for both volume and planar implants. The total nominal dwell times calculated by the Manchester method (e.g., Eqs. 4 and 5) and the planning system can typically be within about 10%. In addition to dose calculations, the reconstruction of implant geometry can yield information about the type of implant, its size and the distance of the implant from the tip of the catheter(s). It can also make use of patient data written into the treatment data file to help validate patient identification and fraction number.

Erroneous plans were simulated and the doses were compared with those calculated for the correct plan using the exemplary system, method and computer-accessible medium, according to an exemplary embodiment of the present disclosure.

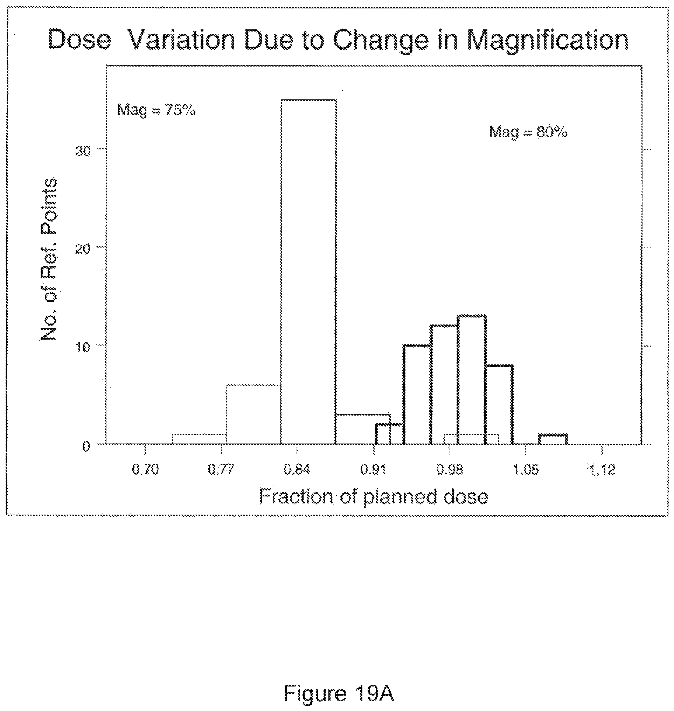

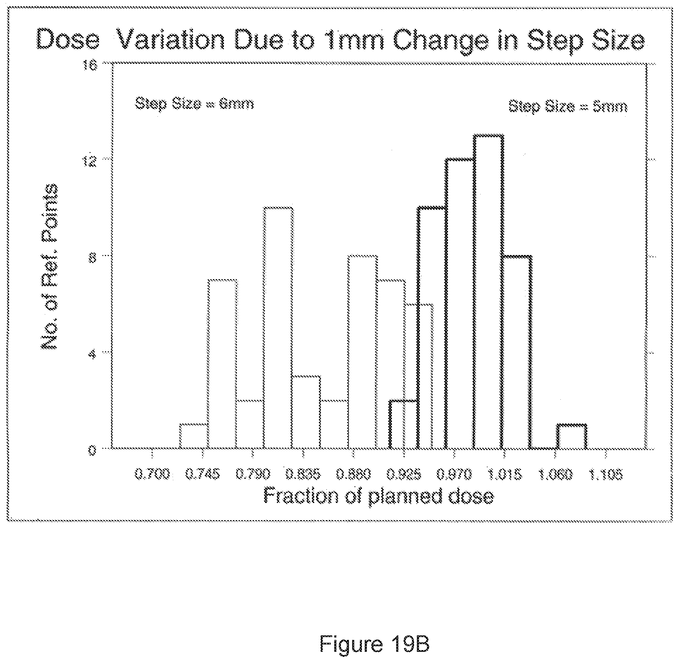

Figures 19A and 19B show exemplary histograms of the ratio of calculated to planned doses for a template-based prostate implant. The calculations were repeated for each plan *(e.g.,* correct or incorrect), to a total of 45 reference points, all at mid gland. In the first example *(see, e.g.,* Figure 19A), dose calculations for the correct plan (e.g., performed at a CT magnification of 0.80) were compared with the dose calculation assuming an error in CT magnification (e.g., assuming the CT magnification should have been 0.75). For example, the dose for the "wrong" plan can be approximately 15% lower than expected, which can be consistent with the squared ratio of magnifications (0.75/0.80)2.

The second example *(see, e.g.,* Figure 19B) shows a comparison of the dose calculation with the correct 5 mm step size and the wrong 6 mm step size. In this case, the "wrong" plan with a 15% decrease in expected dose was consistent with the ratio of implant lengths (0.5/0.6). In the first example, the increase in distance between the reference points and the dwell positions results in the narrowing of the dose range for the "wrong" plan. For the second case, the increase in the range of dose calculated can be caused by non-uniform changes of the distances between individual reference points to adjacent dwell positions. This can be most pronounced for higher doses, for example, for reference points that can be relatively close to the dwell positions. Further, the physical length of the implant, which can be part of the output of the exemplary system, method and computer-accessible medium, can also suggest the presence of an error in the second example. For consistency, all other parameters (e.g., such as dwell time, prescribed dose, etc.) can be kept constant in either example.

***Exemplary Conclusion***

AAPM TG-59 provides a comprehensive discussion of plan evaluation methods as well as an extensive QA checklist, including items for pretreatment review. The exemplary system, method and computer-accessible medium, according to an exemplary embodiment of the present disclosure, can complement and enhance the recommendations of TG-59. It can assist the physicist in *(e.g.,* what can be sometimes a tedious and error prone task of) pretreatment review of various treatment parameters, and provides additional dose verification. It should be understood that an independent dose calculation alone may not be sufficient for treatment planning QA.

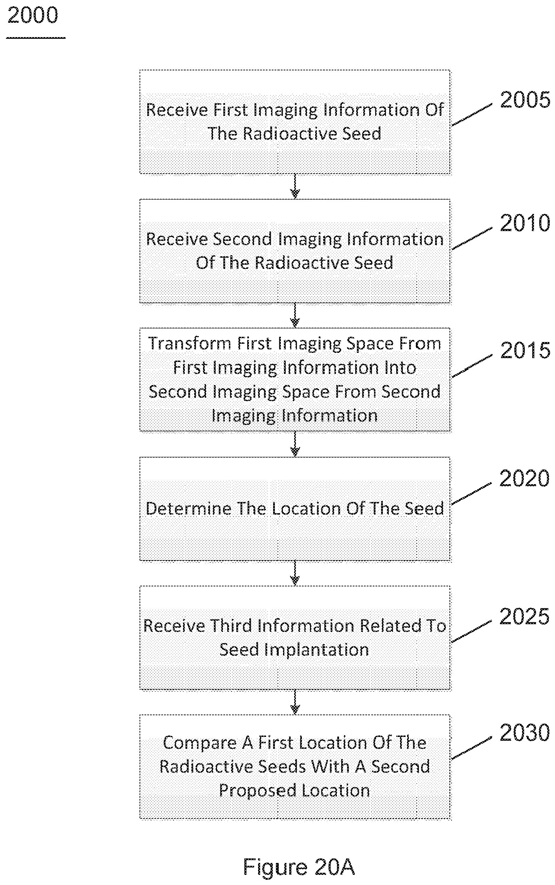

Figure 20A shows an exemplary flow diagram of an exemplary method 2000 for determining the location of a radioactive seed within the body according to an exemplary embodiment of the present disclosure. For example, at procedure 2005, first imaging information of the radioactive seed within the body based on a first imaging modality can be received. At procedure 2010, second imaging information of the radioactive seed within the body based on a second imaging modality can be received. At procedure 2015, the first imaging information from the first imaging space can be transformed to the second imaging space from the second imaging information. At procedure 2020, the location of the seed can be determined based on the first and second imaging information. At procedure 2025, third information related to a seed implantation can be received, and at procedure 2030, a first location of the seed can be compared to a second proposed location of the seed.

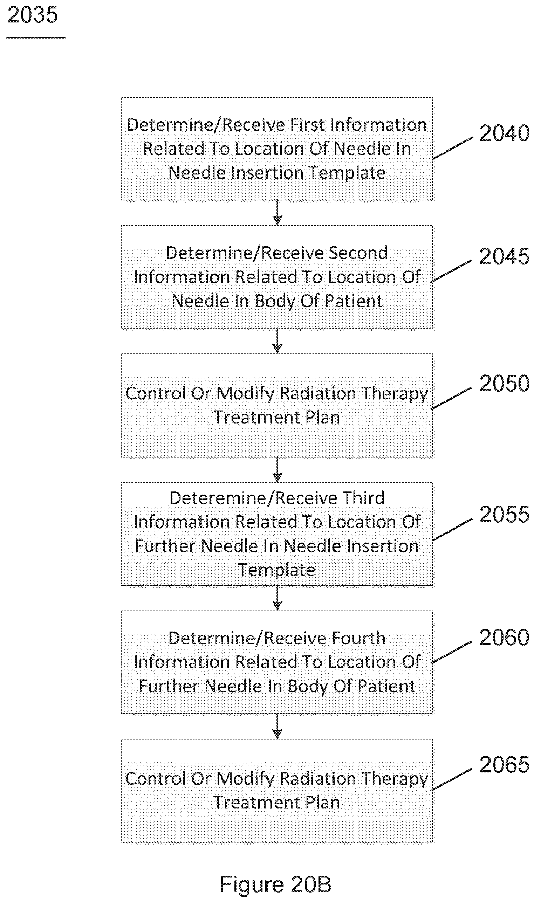

Figure 20B shows an exemplary flow diagram of an exemplary method 2035 for controlling or modifying a radiation therapy treatment plan associated with a patient according to an exemplary embodiment of the present disclosure. For example, at procedure 2040, first information related to a first location of a needle in a needle insertion template can be determined and/or received. At procedure 2045, second information related to a second location of the needle in a body of the patient can be determined and/or received. At procedure 2050, the radiation therapy treatment plan can be controlled or modified based on the first and second information. At procedure 2055, third information related to a third location of a further needle in the needle insertion template can be determined and/or received. At procedure 2060, fourth information related to a fourth location of the further needle in the body of the patient can be determined and/or received. At procedure 2065, the radiation therapy treatment plan can be controlled or modified based on the first, second, third and fourth information.

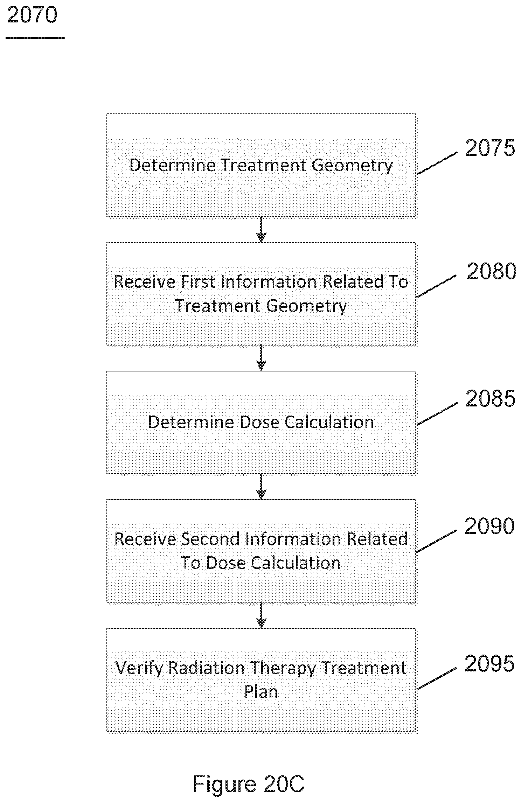

Figure 20C shows an exemplary flow diagram of an exemplary method 2070 for verifying a radiation therapy treatment plan according to an exemplary embodiment of the present disclosure. For example, at procedure 2075, treatment geometry can be determined, which can be received at procedure 2080. At procedure 2085, a dose calculation can be determined, which can be received at procedure 2090. At procedure 2095, the radiation therapy plan can be verified.

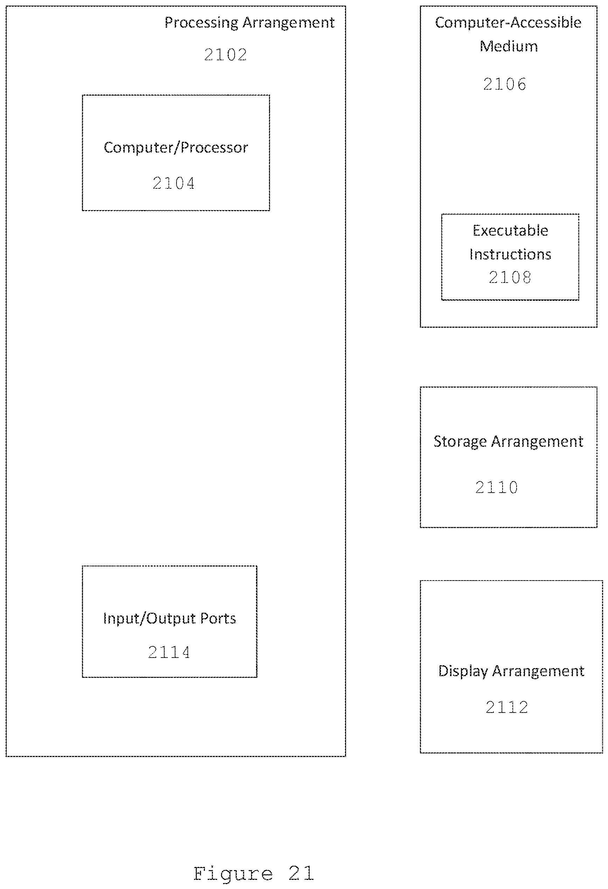

Figure 21 shows a block diagram of an exemplary embodiment of a system according to the present disclosure. For example, exemplary procedures in accordance with the present disclosure described herein can be performed by a processing arrangement and/or a computing arrangement 2102. Such processing/computing arrangement 2102 can be, for example entirely or a part of, or include, but not limited to, a computer/processor 2104 that can include, for example one or more microprocessors, and use instructions stored on a computer-accessible medium (e.g., RAM, ROM, hard drive, or other storage device).

As shown in Figure 21, for example a computer-accessible medium 2106 (e.g., as described herein above, a storage device such as a hard disk, floppy disk, memory stick, CD-ROM, RAM, ROM, etc., or a collection thereof) can be provided (e.g., in communication with the processing arrangement 2102). The computer-accessible medium 2106 can contain executable instructions 2108 thereon. In addition or alternatively, a storage arrangement 2110 can be provided separately from the computer-accessible medium 2106, which can provide the instructions to the processing arrangement 2102 so as to configure the processing arrangement to execute certain exemplary procedures, processes and methods, as described herein above, for example.

Further, the exemplary processing arrangement 2102 can be provided with or include an input/output arrangement 2114, which can include, for example a wired network, a wireless network, the internet, an intranet, a data collection probe, a sensor, etc. As shown in Figure 21, the exemplary processing arrangement 2102 can be in communication with an exemplary display arrangement 2112, which, according to certain exemplary embodiments of the present disclosure, can be a touch-screen configured for inputting information to the processing arrangement in addition to outputting information from the processing arrangement, for example. Further, the exemplary display 2112 and/or a storage arrangement 2110 can be used to display and/or store data in a user-accessible format and/or user-readable format.

The foregoing merely illustrates the principles of the disclosure. Various modifications and alterations to the described embodiments will be apparent to those skilled in the art in view of the teachings herein. It will thus be appreciated that those skilled in the art will be able to devise numerous systems, arrangements, and procedures which, although not explicitly shown or described herein, embody the principles of the disclosure and can be thus within the spirit and scope of the disclosure. Various different exemplary embodiments can be used together with one another, as well as interchangeably therewith, as should be understood by those having ordinary skill in the art. In addition, certain terms used in the present disclosure, including the specification, drawings and claims thereof, can be used synonymously in certain instances, including, but not limited to, for example, data and information. It should be understood that, while these words, and/or other words that can be synonymous to one another, can be used synonymously herein, that there can be instances when such words can be intended to not be used synonymously. Further, to the extent that the prior art knowledge has not been explicitly incorporated by reference herein above, it is explicitly incorporated herein in its entirety. All publications referenced are incorporated herein by reference in their entireties.

## EXEMPLARY REFERENCES

The following references are hereby incorporated by reference in their entirety.

1. US Nuclear Regulatory Commission. 10 CFR 35.2.

2. Kutcher GJ, Coja L, Gillin M, et al. Comprehensive QA for radiation oncology: Report of AAPM Radiation Therapy Committee Task Group No. 40. Med Phys 1994;21:581-618.

3. Kubo HD, Glasgow GP, Pethel TD, et al. High dose-rate brachytherapy treatment delivery: Report of AAPM Radiation Therapy Committee Task Group No. 59. Med Phys 1998;25: 375—403.

4. Ezzell GA. Quality assurance of treatment plans for optimized high dose rate brachytherapy—planar implants. Med Phys 1994;21:659 - 661.

5. Venselaar JLM, Bierhuizen HWJ, Klop R. A method to check treatment time calculations in Ir-192 high-dose-rate volume implants. Med Phys 1995;22:1499 -1500.

6. Rogus RD, Smith MJ, Kubo HD. An equation to QA the total treatment time for single-catheter HDR brachytherapy. Int J Radiat Oncol Biol Phys 1998;40:245-248.

7. Miller AV, Davis MG, Horton JL. A method for verifying treatment times for simple high-dose-rate endobronchial brachytherapy procedures. Med Phys 1996;23:1903-1908.

8. Saw CB, Korb LJ, Darnell B, et al. Independent technique of verifying high-dose rate (HDR) brachytherapy treatment plans. Int J Radiat Oncol Biol Phys 1998;40:747-750.

9. Williamson JF, Ezzell GA, Olch OA, et al. Quality assurance in high dose rate brachytherap. In: Nag S, editor. High dose date remote brachytherapy: A textbook. Armonk, NY: Futura Publishing Company; 1994. p. 147-212.

10. Nag S, Lukas P, Thomas DS, et al. Intraoperative high dose rate remote brachytherapy. In: Nag S, editor. High dose rate remote brachytherapy: A textbook. Armonk, NY: Futura Publishing Company; 1994. p. 427- 445.

11. Anderson LL, Hoffman MR, Harrington PJ, et al. Atlas generation for intraoperative high dose rate brachytherapy. J Brachyther Int 1997;13:333-340.

12. Meisberger L, Keller R, Shalek R. The effective attenuation in water of the gamma rays of gold-198, iridium-192, cesium-137, radium-226 and cobalt-60. Radiology 1986;90:953.

13. Hoskin PJ, Rembowska A. Dosimetry rules for brachytherapy using high dose rate remote afterloading implants. Clin Oncol 1998;10:226 -230.

14. Merredith WJ, editor. Radium dosage. The Manchester System. Edinburgh: Livingston; 1967.

15. International Commission on Radiation Units, and Measurements. Dose and Volume Specification for Reporting Interstitial Therapy. ICRU Report No. 58. Bethesda: ICRU; 1997.

16. Anderson, L. L., M. R. Hoffman, P. J. Harrington, and G. Starkschall. (1997). "Atlas generation for intraoperative high dose rate brachytherapy." J Brachyther Int 13:333-340.

17. Cohen, G. N., H. I. Amols, and M. Zaider. (2000). "An independednt dose-to-point calculation program for the verification of high-dose-rate brachytherapy treatment planning." Int J Radiat Oncol Biol Phys 48(4): 1251-1258.

18. Ezzell, G. A. (1994). "Quality assurance of treatment plans for optimized high dose rate brachytherapy-planar implants." Med Phys 21:659-661.

19. Harrison, L.B., B. D. Minsky, W. E. Enker, B. Mychalczak, J. Guillem, P. B. Paty, L. L. Anderson, C. White, and A. M. Cohen. (1998). "High dose rate intraoperative radiation therapy (HDR-IORT) as part of the management strategy for locally advanced primary and recurrent rectal cancer." Int J Radiat Oncol Biol Phys 42(2):325-330.

20. International Commission on Radiation Units and Measurements (ICRU). Report No. 58. Dose and Volume Specification for Reporting Interstitial Therapy. Bethesda, MD: ICRU, 1997.

21. Kubo, H. D., G. P. Glasgow, T. D. Pethel, B. R. Thomadsen, and J. F. Williamson. (1998). "High dose-rate brachytherapy treatment delivery: Report of the AAPM Radiation Therapy Committee Task Group No. 59." Med Phys 25(4):375-403. Also available as AAPM Report No. 61.

22. Meisberger, L., R. Keller, and R. Shalek. (1986). "The effective attenuation in water of the gamma rays of gold 198, iridium 192, cesium 137, radium 226 and cobalt 60." Radiology 90:953-957.

23. Meredith, W. J., (ed). Radium Dosage. The Manchester System. Edinburgh: Livingston, 1967.

24. Miller, A. V., M. G. Davis, and J. L. Horton. (1996). "A method for verifying treatment times for simple high-doserate endobronchial brachytherapy procedures." Med Phys 23:1903-1908.

25. Nag, S., P. Lukas, D. S. Thomas, and L. B. Harrison. "Intraoperative High Dose Rate Remote Brachytherapy" in High Dose Rate Brachytherapy: A Textbook. S. Nag. Armonk NY: Futura Publishing Company, 1994.

26. Nath, R., L. L. Anderson, J. A. Meli, A. J. Olch, J. A. Stitt, and J. F. Williamson. (1997). "Code of practice for brachytherapy physics: Report of the AAPM Radiation Therapy Committee Task Group No. 56." Med Phys 24(10):1557-1598. Also available as AAPM Report No. 59.

27. Rogus, R. D., M. J. Smith, and H. D. Kubo. (1998). "An equation to QA the total treatment time for single-catheter HDR brachytherapy." Int J Radiat Oncol Biol Phys 40:245-248.

28. Saw, C. B., L. J. Korb, B. Darnell, K. V. Krishna, and D. Ulewicz. (1998). "Independent technique of verifying highdose rate (HDR) brachytherapy treatment plans." Int J Radiat Oncol Biol Phys 40:747-750.

29. Venselaar, J. L., H. W. Bierhuizen, and R. Klop. (1995). "A method to check treatment time calculations in Ir-192 high-dose-rate volume implants." Med Phys 22:1499-1500.

## EMBODIMENTS:

1. A non-transitory computer-accessible medium having stored thereon computer-executable instructions for determining a location of at least one radioactive seed within a body, wherein, when a computer arrangement executes the instructions, the computer arrangement is configured to perform procedures comprising:

receiving first imaging information of the at least one radioactive seed within the body based on a first imaging modality;
receiving second imaging information of the at least one radioactive seed within the body based on a second imaging modality; and
determining the location of the at least one radioactive seed within the body based on the first and second imaging information.

2. The computer-accessible medium of embodiment 1, wherein the first imaging modality is computed tomography.

3. The computer-accessible medium of embodiment 1, wherein the second imaging modality is an ultrasound.

4. The computer-accessible medium of embodiment 3, wherein the ultrasound is a transrectal ultrasound.

5. The computer-accessible medium of embodiment 1, wherein the computer arrangement is further configured to receive third information related to a seed implantation plan for at least one patient.

6. The computer-accessible medium of embodiment 5, wherein the at least one radioactive seed includes a plurality of radioactive seeds, and wherein the computer arrangement is further configured to compare a first location of each radioactive seed of the radioactive seeds in the body of the at least one patient with a second proposed location in the body of the at least one patient for each radioactive seed contained in the third information.

7. The computer-accessible medium of embodiment 1, wherein the first imaging information is in a first imaging space, and the second imaging information is in a second imaging space.

8. The computer-accessible medium of embodiment 7, wherein the computer arrangement is further configured to transform the first imaging information from the first imaging space to the second imaging space.

9. A method for determining a location of at least one radioactive seed within a body, comprising:

receiving first imaging information of the at least one radioactive seed within the body based on a first imaging modality;
receiving second imaging information of the at least one radioactive seed within the body based on a second imaging modality; and
with a specifically-configured computer hardware arrangement, determining the location of the at least one seed within the body based on the first and second imaging information.

10. The method of embodiment 9, wherein the first imaging modality is computed tomography.

11. The method of embodiment 9, wherein the second imaging modality is an ultrasound.

12. The method of embodiment 11, wherein the ultrasound is a transrectal ultrasound.

13. The method of embodiment 9, further comprising receiving third information related to a seed implantation plan for at least one patient.

14. The method of embodiment 13, wherein the at least one radioactive seed includes a plurality of radioactive seeds, and further comprising comparing a first location of each radioactive seed of the radioactive seeds in the body of the at least one patient with a second proposed location in the body of the at least one patient for each radioactive seed contained in the third information.

15. The method of embodiment 9, wherein the first imaging information is in a first imaging space, and the second imaging information is in a second imaging space.

16. The method of embodiment 15, further comprising transforming the first imaging information from the first imaging space to the second imaging space.

17. A system for determining a location of at least one radioactive seed within a body, comprising:
a computer hardware arrangement configured to:

receive first imaging information of the at least one radioactive seed within the body based on a first imaging modality;
receive second imaging information of the at least one radioactive seed within the body based on a second imaging modality; and
determine the location of the at least one seed within the body based on the first and second imaging information.

18. The system of embodiment 17, wherein the first imaging modality is computed tomography.

19. The system of embodiment 17, wherein the second imaging modality is an ultrasound.

20. The system of embodiment 19, wherein the ultrasound is a transrectal ultrasound.

21. The system of embodiment 17, wherein the computer hardware arrangement is further configured to receive third information related to a seed implantation plan for at least one patient.

22. The system of embodiment 21, wherein the at least one radioactive seed includes a plurality of radioactive seeds, and wherein the computer hardware arrangement is further configured to compare a first location of each radioactive seed of the radioactive seeds in the body of the at least one patient with a second proposed location in the body of the at least one patient for each radioactive seed contained in the third information.

23. The system of embodiment 17, wherein the first imaging information is in a first imaging space, and the second imaging information is in a second imaging space.

24. The system of embodiment 23, wherein the computer hardware arrangement is further configured to transform the first imaging information from the first imaging space to the second imaging space.

25. A non-transitory computer-accessible medium having stored thereon computer-executable instructions for controlling or modifying at least one radiation therapy treatment plan associated with a patient, wherein, when a computer arrangement executes the instructions, the computer arrangement is configured to perform procedures comprising:

receiving first information related to a first location of at least one needle in at least one needle insertion template;
receiving second information related to a second location of the at least one needle in a body of the patient; and
controlling or modifying the at least one radiation therapy treatment plan based on the first and second information.

26. The computer-accessible medium of embodiment 25, wherein the computer arrangement is further configured to:

receive third information related to a third location of at least one further needle in the at least one needle insertion template;
receive fourth information related to a fourth location of the at least one further needle in the body of the patient; and
control or modify the at least one radiation therapy treatment plan based on the first, second, third and fourth information.

27. The computer-accessible medium of embodiment 25, wherein the computer arrangement is configured to modify the at least one radiation therapy treatment plan by determining a location of an insertion of at least one further needle in the body of the patient.

28. The computer-accessible medium of embodiment 25, wherein the computer arrangement is further configured to determine the first information by:

receiving third information related to light received by at least one light emitting diode (LED) detector;
receiving fourth information related to an absence of the light at the at least one LED detector; and
determining the first location of the at least one needle in the at least one needle insertion template based on the fourth information.

29. The computer-accessible medium of embodiment 28, wherein the at least one needle insertion template includes a plurality of needle insertion holes, and wherein the at least one LED detector is associated with a particular one of the needle insertion holes.

30. The computer-accessible medium of embodiment 25, wherein the computer arrangement is further configured to determine the second information by:

receiving third information related to at least one ultrasound (US) image without the at least one needle contained therein;
receiving fourth information related to at least one further US image having the at least one needle contained therein; and

determining the second information based on the third and fourth information.

31. The computer-accessible medium of embodiment 30, wherein the computer arrangement is configured to determine the second information by digitally subtracting the third information from the fourth information.

32. The computer-accessible medium of embodiment 25, wherein the at least one needle insertion template includes a plurality of needle insertion holes.

33. The computer-accessible medium of embodiment 25, wherein the computer arrangement is further configured to generate the at least one radiation therapy treatment plan.

34. A method for controlling or modifying at least one radiation therapy treatment plan associated with a patient, comprising:

receiving first information related to a first location of at least one needle in at least one needle insertion template;
receiving second information related to a second location of the at least one needle in a body of the patient; and
using a computer-hardware arrangement, controlling or modifying the at least one radiation therapy treatment plan based on the first and second information.

35. The method of embodiment 34, further comprising:

receiving third information related to a third location of at least one further needle in the at least one needle insertion template;
receiving fourth information related to a fourth location of the at least one further needle in the body of the patient; and
controlling or modifying the at least one radiation therapy treatment plan based on the first, second, third and fourth information.

36. The method of embodiment 34, further comprising modifying the at least one radiation therapy treatment plan by determining a location of an insertion of at least one further needle in the body of the patient.

37. The method of embodiment 34, further comprising determining the first information by:

receiving third information related to light received by at least one light emitting diode (LED) detector;
receiving fourth information related to an absence of the light at the at least one LED detector; and
determining the first location of the at least one needle in the at least one needle insertion template based on the fourth information.

38. The method of embodiment 37, wherein the at least one needle insertion template includes a plurality of needle insertion holes, and wherein the at least one LED detector is associated with a particular one of the needle insertion holes.

39. The method of embodiment 34, further comprising determining the second information by:

receiving third information related to at least one ultrasound (US) image without the at least one needle contained therein;
receiving fourth information related to at least one further US image having the at least one needle contained therein; and
determining the second information based on the third and fourth information.

40. The method of embodiment 39, further comprising determining the second information by digitally subtracting the third information from the fourth information.

41. The method of embodiment 34, wherein the at least one needle insertion template includes a plurality of needle insertion holes.

42. The method of embodiment 34, further comprising generating the at least one radiation therapy treatment plan.

43. A system for controlling or modifying at least one radiation therapy treatment plan associated with a patient, comprising:
a computer hardware arrangement configured to:

receiving first information related to a first location of at least one needle in at least one needle insertion template;
receiving second information related to a second location of the at least one needle in a body of the patient; and
controlling or modifying the at least one radiation therapy treatment plan based on the first and second information.

44. The system of embodiment 43, wherein the computer hardware arrangement is further configured to:

receive third information related to a third location of at least one further needle in the at least one needle insertion template;
receive fourth information related to a fourth location of the at least one further needle in the body of the patient; and
control or modify the at least one radiation therapy treatment plan based on the first, second, third and fourth information.

45. The system of embodiment 43, wherein the computer hardware arrangement is configured to modify the at least one radiation therapy treatment plan by determining a location of an insertion of at least one further needle in the body of the patient.

46. The system of embodiment 43, wherein the computer hardware arrangement is further configured to determine the first information by:

receiving third information related to light received by at least one light emitting diode (LED) detector;
receiving fourth information related to an absence of the light at the at least one LED detector; and
determining the first location of the at least one needle in the at least one needle insertion template based on the fourth information.

47. The system of embodiment 46, wherein the at least one needle insertion template includes a plurality of needle insertion holes, and wherein the at least one LED detector is associated with a particular one of the needle insertion holes.

48. The system of embodiment 43, wherein the computer hardware arrangement is further configured to determine the second information by:

receiving third information related to at least one ultrasound (US) image without the at least one needle contained therein;
receiving fourth information related to at least one further US image having the at least one needle contained therein; and
determining the second information based on the third and fourth information.

49. The system of embodiment 48, wherein the computer hardware arrangement is configured to determine the second information by digitally subtracting the third information from the fourth information.

50. The system of embodiment 43, wherein the at least one needle insertion template includes a plurality of needle insertion holes.

51. The system of embodiment 43, wherein the computer hardware arrangement is further configured to generate the at least one radiation therapy treatment plan.

52. A non-transitory computer-accessible medium having stored thereon computer-executable instructions for verifying at least one radiation therapy treatment plan, wherein, when a computer arrangement executes the instructions, the computer arrangement is configured to perform procedures comprising:

receiving first information related to at least one treatment geometry associated with the at least one radiation therapy treatment plan;

receiving second information related to at least one dose calculation based on the at least one treatment geometry; and

verifying the at least one radiation therapy treatment plan based on the first and second information.

53. The computer-accessible medium of embodiment 52, wherein the computer arrangement is further configured to determine the at least one treatment geometry.

54. The computer-accessible medium of embodiment 53, wherein computer arrangement is configured to determine the at least one treatment geometry based on a plurality of dwell positions.

55. The computer-accessible medium of embodiment 54, wherein the dwell positions are correct dwell positions.

56. The computer-accessible medium of embodiment 54, wherein each of the dwell positions includes a nominal dwell time.

57. The computer-accessible medium of embodiment 56, wherein each of the dwell positions further includes a set of coordinates in a patient's coordinate space.

58. The computer-accessible medium of embodiment 57, wherein the set of coordinates is defined by a plurality of active channels.

59. The computer-accessible medium of embodiment 57, wherein the computer arrangement is further configured to approximate the set of coordinates using a user input.

60. The computer-accessible medium of embodiment 52, wherein the computer arrangement is further configured to determine the at least one dose calculation.

61. The computer-accessible medium of embodiment 60, wherein the at least one dose calculation includes at least one dose-to-point calculation.

62. The computer-accessible medium of embodiment 60, wherein the at least one dose calculation includes a summation of dose contributions from all dwell positions based on a two-dimensional dose table.

63. A method for verifying at least one radiation therapy treatment plan, comprising:

receiving first information related to at least one treatment geometry associated with the at least one radiation therapy treatment plan;
receiving second information related to at least one dose calculation based on the at least one treatment geometry; and
with a specifically-configured computer hardware arrangement, verifying the at least one radiation therapy treatment plan based on the first and second information.

64. The method of embodiment 63, further comprising determining the at least one treatment geometry.

65. The method of embodiment 64, further comprising determining the at least one treatment geometry based on a plurality of dwell positions.

66. The method of embodiment 65, wherein the dwell positions are correct dwell positions.

67. The method of embodiment 65, wherein each of the dwell positions includes a nominal dwell time.

68. The method of embodiment 67, wherein each of the dwell positions further includes a set of coordinates in a patient's coordinate space.

69. The method of embodiment 68, wherein the set of coordinates is defined by a plurality of active channels.

70. The method of embodiment 68, further comprising approximating the set of coordinates using a user input.

71. The method of embodiment 63, further comprising determining the at least one dose calculation.

72. The method of embodiment 71, wherein the at least one dose calculation includes at least one dose-to-point calculation.

73. The method of embodiment 71, wherein the at least one dose calculation includes a summation of dose contributions from all dwell positions based on a two-dimensional dose table.

74. A system for verifying at least one radiation therapy treatment plan, comprising:
a computer hardware arrangement configured to:

receive first information related to at least one treatment geometry associated with the at least one radiation therapy treatment plan;
receive second information related to at least one dose calculation based on the at least one treatment geometry; and
verify the at least one radiation therapy treatment plan based on the first and second information.

75. The system of embodiment 74, wherein the computer hardware arrangement is further configured to determine the at least one treatment geometry.

76. The system of embodiment 75, wherein computer hardware arrangement is configured to determine the at least one treatment geometry based on a plurality of dwell positions.

77. The system of embodiment 76, wherein the dwell positions are correct dwell positions.

78. The system of embodiment 76, wherein each of the dwell positions includes a nominal dwell time.

79. The system of embodiment 78, wherein each of the dwell positions further includes a set of coordinates in a patient's coordinate space.

80. The system of embodiment 79, wherein the set of coordinates is defined by a plurality of active channels.

81. The system of embodiment 79, wherein the computer hardware arrangement is further configured to approximate the set of coordinates using a user input.

82. The system of embodiment 74, wherein the computer hardware arrangement is further configured to determine the at least one dose calculation.

83. The system of embodiment 82, wherein the at least one dose calculation includes at least one dose-to-point calculation.

84. The system of embodiment 82, wherein the at least one dose calculation includes a summation of dose contributions from all dwell positions based on a two-dimensional dose table.

**Claims**

1. A non-transitory computer-accessible medium having stored thereon computer-executable instructions for controlling or modifying at least one radiation therapy treatment plan associated with a patient, wherein, when a computer arrangement executes the instructions, the computer arrangement is configured to perform procedures comprising:

receiving first information related to a first location of at least one needle in at least one needle insertion template;
receiving second information related to a second location of the at least one needle in a body of the patient; and
controlling or modifying the at least one radiation therapy treatment plan based on the first and second information.

2. The computer-accessible medium of claim 1, wherein the computer arrangement is further configured to:

receive third information related to a third location of at least one further needle in the at least one needle insertion template;

receive fourth information related to a fourth location of the at least one further needle in the body of the patient; and

control or modify the at least one radiation therapy treatment plan based on the first, second, third and fourth information.

3. The computer-accessible medium of claim 1 or 2, wherein the computer arrangement is configured to modify the at least one radiation therapy treatment plan by determining a location of an insertion of at least one further needle in the body of the patient.

4. The computer-accessible medium of any one of the preceding claims, wherein the computer arrangement is further configured to determine the first information by:

receiving third information related to light received by at least one light emitting diode, LED, detector;

receiving fourth information related to an absence of the light at the at least one LED detector; and

determining the first location of the at least one needle in the at least one needle insertion template based on the fourth information.

5. The computer-accessible medium of claim 4, wherein the at least one needle insertion template includes a plurality of needle insertion holes, and wherein the at least one LED detector is associated with a particular one of the needle insertion holes.

6. The computer-accessible medium of any one of the preceding claims, wherein the computer arrangement is further configured to determine the second information by:

receiving third information related to at least one ultrasound, US, image without the at least one needle contained therein;

receiving fourth information related to at least one further US image having the at least one needle contained therein; and

determining the second information based on the third and fourth information.

7. The computer-accessible medium of claim 6, wherein the computer arrangement is configured to determine the second information by digitally subtracting the third information from the fourth information.

8. The computer-accessible medium of any one of the preceding claims, wherein the at least one needle insertion template includes a plurality of needle insertion holes.

9. The computer-accessible medium of any one of the preceding claims, wherein the computer arrangement is further configured to generate the at least one radiation therapy treatment plan.

10. A method for controlling or modifying at least one radiation therapy treatment plan associated with a patient, comprising:

receiving first information related to a first location of at least one needle in at least one needle insertion template;

receiving second information related to a second location of the at least one needle in a body of the patient; and

using a computer-hardware arrangement, controlling or modifying the at least one radiation therapy treatment plan based on the first and second information.

11. The method of claim 10, further comprising:

receiving third information related to a third location of at least one further needle in the at least one needle insertion template;

receiving fourth information related to a fourth location of the at least one further needle in the body of the patient; and

controlling or modifying the at least one radiation therapy treatment plan based on the first, second, third and fourth information; and/or

modifying the at least one radiation therapy treatment plan by determining a location of an insertion of at least one further needle in the body of the patient.

12. The method of claim 10 or 11, further comprising determining the first information by:

receiving third information related to light received by at least one light emitting diode, LED, detector;
receiving fourth information related to an absence of the light at the at least one LED detector; and
determining the first location of the at least one needle in the at least one needle insertion template based on the fourth information,
wherein the at least one needle insertion template includes a plurality of needle insertion holes, and wherein the at least one LED detector is associated with a particular one of the needle insertion holes.

13. The method of any one of claims 10 to 12, further comprising determining the second information by:

receiving third information related to at least one ultrasound, US, image without the at least one needle contained therein;
receiving fourth information related to at least one further US image having the at least one needle contained therein;
determining the second information based on the third and fourth information; and/or
determining the second information by digitally subtracting the third information from the fourth information.

14. A system for controlling or modifying at least one radiation therapy treatment plan associated with a patient, comprising:
a computer hardware arrangement configured to:

receiving first information related to a first location of at least one needle in at least one needle insertion template;
receiving second information related to a second location of the at least one needle in a body of the patient; and
controlling or modifying the at least one radiation therapy treatment plan based on the first and second information.

Figure 1

EP 3 939 512 A1

Figure 2

Figure 3A

Figure 3B

Figure 4A

Figure 4B

Figure 4C

Figure 5A

Figure 5B

Figure 5C

EP 3 939 512 A1

Figure 6

Figure 7

Coronal                  Sagittal

Figure 9

Figure 8A            Figure 8B            Figure 8C

Figure 8D

coronal            sagittal

Figure 9

1005

Figure 10

Figure 11

1205

Figure 12

Figure 13

(HOSPITAL NAME)
DEPARTMENT OF RADIATION ONCOLOGY
**HDR INTRA-OPERATIVE BRACHYTHERAPY PLANNING WORKSHEET**

PATIENT NAME:_____ MRN/ID: __-____-____Date____/____/20___

RADIATION ONCOLOGIST/RESIDENT:_____;TREATMENT SITE:_____

CHANNELS[1]___x POSITIONS[1]__;AREA[1](CM): W__xL ____; DOSE[1]_____Gy

Treatment positions ▶1-distal (tip of catheter), n=proximal

1. These parameters refer to the total treatment area. Regions of dose (de)escalation should be delineated graphically and the prescribed dose for each region written on the graph.

## Figure 14

Figure 15

Figure 16

Figure 17

Bladder

Prostate

Needles
Template

Y

$Y_{tip,6}$

$Y_{cut}$

$(X_{cut,6}; Z_{cut,6})$

Z

X

$\theta$

1 2 3 4 5 6 7

**Figure 18**

Figure 19A

Figure 19B

2000

Receive First Imaging Information Of The Radioactive Seed — 2005

Receive Second Imaging Information Of The Radioactive Seed — 2010

Transform First Imaging Space From First Imaging Information Into Second Imaging Space From Second Imaging Information — 2015

Determine The Location Of The Seed — 2020

Receive Third Information Related To Seed Implantation — 2025

Compare A First Location Of The Radioactive Seeds With A Second Proposed Location — 2030

Figure 20A

2035

```
┌─────────────────────────────────┐
│ Determine/Receive First         │        2040
│ Information Related To Location  │
│ Of Needle In Needle Insertion   │
│ Template                        │
└─────────────────────────────────┘
             │
             ▼
┌─────────────────────────────────┐
│ Determine/Receive Second        │        2045
│ Information Related To Location  │
│ Of Needle In Body Of Patient    │
└─────────────────────────────────┘
             │
             ▼
┌─────────────────────────────────┐
│ Control Or Modify Radiation      │       2050
│ Therapy Treatment Plan           │
└─────────────────────────────────┘
             │
             ▼
┌─────────────────────────────────┐
│ Deteremine/Receive Third        │        2055
│ Information Related To Location  │
│ Of Further Needle In Needle      │
│ Insertion Template              │
└─────────────────────────────────┘
             │
             ▼
┌─────────────────────────────────┐
│ Determine/Receive Fourth        │        2060
│ Information Related To Location  │
│ Of Further Needle In Body Of     │
│ Patient                         │
└─────────────────────────────────┘
             │
             ▼
┌─────────────────────────────────┐
│ Control Or Modify Radiation      │       2065
│ Therapy Treatment Plan           │
└─────────────────────────────────┘
```

Figure 20B

2070

```
┌─────────────────────────────────┐
│                                 │
│   Determine Treatment Geometry  │ ──── 2075
│                                 │
└─────────────────────────────────┘
                 │
                 ▼
┌─────────────────────────────────┐
│  Receive First Information Related To  │ ──── 2080
│       Treatment Geometry        │
└─────────────────────────────────┘
                 │
                 ▼
┌─────────────────────────────────┐
│                                 │
│    Determine Dose Calculation   │ ──── 2085
│                                 │
└─────────────────────────────────┘
                 │
                 ▼
┌─────────────────────────────────┐
│  Receive Second Information Related  │ ──── 2090
│       To Dose Calculation       │
└─────────────────────────────────┘
                 │
                 ▼
┌─────────────────────────────────┐
│  Verify Radiation Therapy Treatment  │ ──── 2095
│              Plan               │
└─────────────────────────────────┘
```

Figure 20C

Processing Arrangement

2102

Computer/Processor

2104

Computer-Accessible Medium

2106

Executable Instructions

2108

Storage Arrangement

2110

Input/Output Ports

2114

Display Arrangement

2112

Figure 21

Europäisches
Patentamt

European
Patent Office

Office européen
des brevets

# EUROPEAN SEARCH REPORT

Application Number

EP 21 17 2134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/001551 A1 (KONINKL PHILIPS ELECTRONICS NV [NL]; BINNEKAMP DIRK [US]; GUTIERREZ LU) 5 January 2012 (2012-01-05) * abstract * * page 9, line 22 – page 11, line 2 * * figure 2 * | 1-14 | INV. A61B6/12 A61B8/12 A61N5/10 A61B6/03 ADD. A61N5/00 A61B18/00 |
| A | US 2004/092786 A1 (ZAIDER MARCO [US] ET AL) 13 May 2004 (2004-05-13) * the whole document * | 1-14 | |
| A | US 2009/014015 A1 (TUTAR ISMAIL [US] ET AL) 15 January 2009 (2009-01-15) * abstract * * paragraph [0018] * * paragraph [0046] – paragraph [0080] * * figures 1,3-4 * | 1-14 | |
| A | US 2010/239144 A1 (FICHTINGER GABOR [CA] ET AL) 23 September 2010 (2010-09-23) * the whole document * | 1-14 | |
| A | US 2004/049109 A1 (THORNTON KENNETH B [US]) 11 March 2004 (2004-03-11) * abstract * * paragraph [0032] – paragraph [0065] * * figures 1-5 * | 1-14 | TECHNICAL FIELDS SEARCHED (IPC) A61B A61N |
| A | US 2011/313285 A1 (FALLAVOLLITA PASCAL [DE] ET AL) 22 December 2011 (2011-12-22) * abstract * * paragraph [0021] – paragraph [0055]; figures 1-2 * | 1-14 | |
| A | US 2010/198063 A1 (HUBER JENNIFER S [US] ET AL) 5 August 2010 (2010-08-05) * the whole document * | 1-14 | |

The present search report has been drawn up for all claims

| Place of search | Date of completion of the search | Examiner |
|---|---|---|
| Munich | 10 December 2021 | Artikis, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 3 939 512 A1

## ANNEX TO THE EUROPEAN SEARCH REPORT
## ON EUROPEAN PATENT APPLICATION NO.

EP 21 17 2134

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2021

| Patent document cited in search report | | Publication date | Patent family member(s) | | Publication date |
|---|---|---|---|---|---|
| WO 2012001551 | A1 | 05-01-2012 | CN | 102971048 A | 13-03-2013 |
| | | | EP | 2588194 A1 | 08-05-2013 |
| | | | TW | 201210645 A | 16-03-2012 |
| | | | US | 2013102891 A1 | 25-04-2013 |
| | | | WO | 2012001551 A1 | 05-01-2012 |
| US 2004092786 | A1 | 13-05-2004 | NONE | | |
| US 2009014015 | A1 | 15-01-2009 | NONE | | |
| US 2010239144 | A1 | 23-09-2010 | CA | 2655001 A1 | 20-08-2010 |
| | | | CA | 2693740 A1 | 20-08-2010 |
| | | | US | 2010239144 A1 | 23-09-2010 |
| US 2004049109 | A1 | 11-03-2004 | AT | 485871 T | 15-11-2010 |
| | | | AU | 2003296969 A1 | 23-08-2004 |
| | | | EP | 1596701 A2 | 23-11-2005 |
| | | | US | 2004049109 A1 | 11-03-2004 |
| | | | WO | 2004066807 A2 | 12-08-2004 |
| US 2011313285 | A1 | 22-12-2011 | CA | 2743937 A1 | 22-12-2011 |
| | | | US | 2011313285 A1 | 22-12-2011 |
| US 2010198063 | A1 | 05-08-2010 | US | 2010198063 A1 | 05-08-2010 |
| | | | WO | 2009009223 A2 | 15-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62170219

**Non-patent literature cited in the description**

- **KUTCHER GJ ; COJA L ; GILLIN M et al.** Comprehensive QA for radiation oncology: Report of AAPM Radiation Therapy Committee Task Group No. 40. *Med Phys,* 1994, vol. 21, 581-618
- **KUBO HD ; GLASGOW GP ; PETHEL TD et al.** High dose-rate brachytherapy treatment delivery: Report of AAPM Radiation Therapy Committee Task Group No. 59. *Med Phys,* 1998, vol. 25, 375-403
- **EZZELL GA.** Quality assurance of treatment plans for optimized high dose rate brachytherapy—planar implants. *Med Phys,* 1994, vol. 21, 659-661
- **VENSELAAR JLM ; BIERHUIZEN HWJ ; KLOP R.** A method to check treatment time calculations in Ir-192 high-dose-rate volume implants. *Med Phys,* 1995, vol. 22, 1499-1500
- **ROGUS RD ; SMITH MJ ; KUBO HD.** An equation to QA the total treatment time for single-catheter HDR brachytherapy. *Int J Radiat Oncol Biol Phys,* 1998, vol. 40, 245-248
- **MILLER AV ; DAVIS MG ; HORTON JL.** A method for verifying treatment times for simple high-dose-rate endobronchial brachytherapy procedures. *Med Phys,* 1996, vol. 23, 1903-1908
- **SAW CB ; KORB LJ ; DARNELL B et al.** Independent technique of verifying high-dose rate (HDR) brachytherapy treatment plans. *Int J Radiat Oncol Biol Phys,* 1998, vol. 40, 747-750
- Quality assurance in high dose rate brachytherap. **WILLIAMSON JF ; EZZELL GA ; OLCH OA et al.** High dose date remote brachytherapy: A textbook. Futura Publishing Company, 1994, 147-212
- Intraoperative high dose rate remote brachytherapy. **NAG S ; LUKAS P ; THOMAS DS et al.** High dose rate remote brachytherapy: A textbook. Futura Publishing Company, 1994, 427-445
- **ANDERSON LL ; HOFFMAN MR ; HARRINGTON PJ et al.** Atlas generation for intraoperative high dose rate brachytherapy. *J Brachyther Int,* 1997, vol. 13, 333-340
- **MEISBERGER L ; KELLER R ; SHALEK R.** The effective attenuation in water of the gamma rays of gold-198, iridium-192, cesium-137, radium-226 and cobalt-60. *Radiology,* 1986, vol. 90, 953

- **HOSKIN PJ ; REMBOWSKA A.** Dosimetry rules for brachytherapy using high dose rate remote afterloading implants. *Clin Oncol,* 1998, vol. 10, 226-230
- Radium dosage. The Manchester System. 1967
- International Commission on Radiation Units, and Measurements. Dose and Volume Specification for Reporting Interstitial Therapy. ICRU, 1997
- **ANDERSON, L. L. ; M. R. HOFFMAN ; P. J. HARRINGTON ; G. STARKSCHALL.** Atlas generation for intraoperative high dose rate brachytherapy. *J Brachyther Int,* 1997, vol. 13, 333-340
- **COHEN, G. N. ; H. I. AMOLS ; M. ZAIDER.** An independednt dose-to-point calculation program for the verification of high-dose-rate brachytherapy treatment planning. *Int J Radiat Oncol Biol Phys,* 2000, vol. 48 (4), 1251-1258
- **EZZELL, G. A.** Quality assurance of treatment plans for optimized high dose rate brachytherapy-planar implants. *Med Phys,* 1994, vol. 21, 659-661
- **HARRISON, L.B. ; B. D. MINSKY ; W. E. ENKER ; B. MYCHALCZAK ; J. GUILLEM ; P. B. PATY ; L. L. ANDERSON ; C. WHITE ; A. M. COHEN.** High dose rate intraoperative radiation therapy (HDR-IORT) as part of the management strategy for locally advanced primary and recurrent rectal cancer. *Int J Radiat Oncol Biol Phys,* 1998, vol. 42 (2), 325-330
- Dose and Volume Specification for Reporting Interstitial Therapy. ICRU, 1997
- **KUBO, H. D. ; G. P. GLASGOW ; T. D. PETHEL ; B. R. THOMADSEN ; J. F. WILLIAMSON.** High dose-rate brachytherapy treatment delivery: Report of the AAPM Radiation Therapy Committee Task Group No. 59. *Med Phys,* 1998, vol. 25 (4), 375-403
- **MEISBERGER, L. ; R. KELLER ; R. SHALEK.** The effective attenuation in water of the gamma rays of gold 198, iridium 192, cesium 137, radium 226 and cobalt 60. *Radiology,* 1986, vol. 90, 953-957
- Radium Dosage. The Manchester System. 1967

- **MILLER, A. V. ; M. G. DAVIS ; J. L. HORTON.** A method for verifying treatment times for simple high-doserate endobronchial brachytherapy procedures. *Med Phys,* 1996, vol. 23, 1903-1908
- Intraoperative High Dose Rate Remote Brachytherapy. **NAG, S. ; P. LUKAS ; D. S. THOMAS ; L. B. HARRISON ; S. NAG.** High Dose Rate Brachytherapy: A Textbook. Futura Publishing Company, 1994
- **NATH, R. ; L. L. ANDERSON ; J. A. MELI ; A. J. OLCH ; J. A. STITT ; J. F. WILLIAMSON.** Code of practice for brachytherapy physics: Report of the AAPM Radiation Therapy Committee Task Group No. 56. *Med Phys,* 1997, vol. 24 (10), 1557-1598
- **ROGUS, R. D. ; M. J. SMITH ; H. D. KUBO.** An equation to QA the total treatment time for single-catheter HDR brachytherapy. *Int J Radiat Oncol Biol Phys,* 1998, vol. 40, 245-248
- **SAW, C. B. ; L. J. KORB ; B. DARNELL ; K. V. KRISHNA ; D. ULEWICZ.** Independent technique of verifying highdose rate (HDR) brachytherapy treatment plans. *Int J Radiat Oncol Biol Phys,* 1998, vol. 40, 747-750
- **VENSELAAR, J. L. ; H. W. BIERHUIZEN ; R. KLOP.** A method to check treatment time calculations in Ir-192 high-dose-rate volume implants. *Med Phys,* 1995, vol. 22, 1499-1500